# EUROPÄISCHE PATENTSCHRIFT

(11) **EP 0 706 523 B1**
(45) Veröffentlichungstag und Bekanntmachung des Hinweises auf die Patenterteilung: **24.01.2001**
(21) Anmeldenummer: 94921630.3
(22) Anmeldetag: 28.06.1994
(51) Int. Cl.: C07D 401/12, C07D 401/14, C07D 417/14, A61K 31/415, A61K 31/44

(54) **SUBSTITUIERTE ARYLTHIOALKYLTHIOPYRIDINE**
SUBSTITUTED ARYLTHIOALKYLTHIOPYRIDINES
ARYLTHIOALKYLTHIOPYRIDINES SUBSTITUEES

(30) Priorität: 29.06.1993 CH 199593
(43) Veröffentlichungstag der Anmeldung: 17.04.1996
(73) Patentinhaber: Byk Gulden Lomberg Chemische Fabrik GmbH, 78467 Konstanz (DE)
(72) Erfinder: KOHL, Bernhard, D-78465 Konstanz (DE); GRUNDLER, Gerhard, D-78464 Konstanz (DE); SENN-BILFINGER, Jörg, D-78464 Konstanz (DE); OPFERKUCH, Wolfgang, D-44801 Bochum (DE)
(86) Internationale Anmeldenummer: EP9402098
(87) Internationale Veröffentlichungsnummer: WO9501351

(56) Entgegenhaltungen:
- EP-A- 0 150 586
- EP-A- 0 268 956
- EP-A- 0 382 489
- EP-A- 0 567 643
- CHEMICAL ABSTRACTS, vol. 115, no. 22, 2. Dezember 1991, Columbus, Ohio, US; abstract no. 239727x, T. KAWAKITA ET AL. 'Bactericides containing benzimidazoles for Campylobacter.' Seite 498 ;Spalte 2 ; & JP,A,03 048 680 (YOSHITOMI PHARMACEUTICAL INDUSTRIES. LTD.) 1. März 1991

## Beschreibung

### Anwendungsgebiet der Erfindung

Die Erfindung betrifft Verbindungen, die in der pharmazeutischen Industrie als Wirkstoffe für die Herstellung von Arzneimitteln verwendet werden sollen.

### Bekannter technischer Hintergrund

In der europäischen Patentanmeldung 150 586 werden 2-(Pyridylmethylthio- bzw. -sulfinyl)-benzimidazole offenbart, die im Pyridinteil des Moleküls in 4-Position unter anderem durch Alkylthio- oder Arylthioreste substituiert sein können. Für die beschriebenen Verbindungen wird eine langanhaltende Magensäuresekretionshemmung angegeben. - In der internationalen Patentanmeldung WO89/03830 ist beschrieben, daß sich dieselben, sowie weitere strukturähnliche Verbindungen zur Behandlung der Osteoporose eignen sollen. - In der internationalen Patentanmeldung WO92/12976 werden auf bestimmte Weise substituierte 2-(Pyridylmethylthio- bzw. -sulfinyl)-benzimidazole beschrieben, die gegen Helicobacter-Bakterien wirksam sein sollen und für die weiterhin offenbart ist, daß sie für die Verhütung und Behandlung einer ganzen Reihe von Erkrankungen des Magens geeignet sein sollen.

### Beschreibung der Erfindung

Gegenstand der Erfindung sind Verbindungen der Formel I (siehe beigefügtes Formelblatt), worin
- R1: Wasserstoff, 1-4C-Alkyl oder 1-4C-Alkoxy bedeutet,
- R2: Wasserstoff, 1-4C-Alkyl, 1-4C-Alkoxy, Halogen, Trifluormethyl, ganz oder überwiegend durch Fluor substituiertes 1-4C-Alkoxy, Chlordifluormethoxy, 2-Chlor-1,1,2-trifluorethoxy oder gemeinsam mit R3 gewünschtenfalls ganz oder teilweise durch Fluor substituiertes 1-2C-Alkylendioxy oder Chlortrifluorethylendioxy bedeutet,
- R3: Wasserstoff, ganz oder überwiegend durch Fluor substituiertes 1-4C-Alkoxy, Chlordifluormethoxy, 2-Chlor-1,1,2-trifluorethoxy oder gemeinsam mit R2 gewünschtenfalls ganz oder teilweise durch Fluor substituiertes 1-2C-Alkylendioxy oder Chlortrifluorethylendioxy bedeutet,
- R4: Wasserstoff oder 1-4C-Alkyl bedeutet,
- R5: Wasserstoff, 1-4C-Alkyl oder 1-4C-Alkoxy bedeutet,
- R6: einen durch R8 und R9 substituierten Cyclus oder Bicyclus bedeutet, der ausgewählt ist aus der Gruppe bestehend aus Benzol, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin und Benzimidazol,
- R7: Wasserstoff, 1-4C-Alkyl oder 1-4C-Alkoxy bedeutet,
- R8: Wasserstoff, 1-4C-Alkyl, Hydroxy, 1-4C-Alkoxy, Halogen, Nitro, Carboxy, 1-4C-Alkoxycarbonyl, Guanidino, durch R10 substituiertes 1-4C-Alkyl oder -N(R11)R12 bedeutet,
- R9: Wasserstoff, 1-4C-Alkyl, 1-4C-Alkoxy, Fluor oder Trifluormethyl bedeutet,
- R10: Hydroxy, 1-4C-Alkoxy, Carboxy, 1-4C-Alkoxycarbonyl oder -N(R11)R12 bedeutet, wobei
- R11: Wasserstoff, 1-4C-Alkyl oder -CO-R13 und
- R12: Wasserstoff oder 1-4C-Alkyl bedeutet, oder wobei
- R11 und R12: zusammen und unter Einschluß des Stickstoffatoms, an das beide gebunden sind, einen Piperidino- oder Morpholinorest darstellen,
- R13: Wasserstoff, 1-4C-Alkyl oder 1-4C-Alkoxy bedeutet,
- m: eine Zahl von 2 bis 7 bedeutet,
- n: die Zahl 0 oder 1 bedeutet,
- p: die Zahl 0 oder 1 bedeutet und
- q: die Zahl 0 oder 1 bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

Halogen im Sinne der vorliegenden Erfindung ist Brom, Chlor und Fluor.

Als ganz oder überwiegend durch Fluor substituiertes 1-4C-Alkoxy seien beispielsweise der 1,2,2-Trifluorethoxy, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als gewünschtenfalls ganz oder teilweise durch Fluor substituiertes 1-2C-Alkylendioxy seien beispielsweise der Methylendioxy- (-O-CH₂-O-), der Ethylendioxy- (-O-CH₂-CH₂-O-), der 1,1-Difluorethylendioxy- (-O-CF₂-CH₂-O-), der 1,1,2,2-Tetrafluorethylendioxy- (-O-CF₂-CF₂-O-) und insbesondere der Difluormethylendioxy- (-O-CF₂-O-) und der 1,1,2-Trifluorethylendioxyrest (-O-CF₂-CHF-O-) genannt.

Wenn R2 und R3 gemeinsam gewünschtenfalls ganz oder teilweise durch Fluor substituiertes 1-2C-Alkylendioxy oder Chlortrifluorethylendioxy bedeuten, so sind die Substituenten R2 und R3 in Nachbarpositionen - bevorzugt an den Positionen 5 und 6 - am Benzoteil des Benzimidazolringes gebunden.

Die Gruppe -S(0)q- ist an ein Kohlenstoffatom des betreffenden Cyclus bzw. Bicyclus R6 angebunden, so daß als Reste R6 beispielsweise genannt seien die Reste: Phenyl, 2-Furyl, 3-Furyl, 2-Thienyl, 3-Thienyl, 3-Pyrrolyl, 2-Oxazolyl, 4-Oxazolyl, 4-Isoxazolyl, 5-Isoxazolyl, 2-Thiazolyl, 3-Isothiazolyl, 2-Imidazolyl, 3-Pyrazolyl, 4-Pyrazolyl, 1,2,3-Triazol-4-yl, 1,2,5-Thiadiazol-4-yl, 1,2,4-Triazol-3-yl, Tetrazol-5-yl, 1,3,4-Thiadiazol-2-yl, 1,2,3-Thiadiazol-4-yl, 1,3,4-Oxadiazol-2-yl, 2-Pyridyl, 4-Pyridyl, 2-Pyrimidinyl und 2-Benzimidazolyl.

Die Substituenten R8 und gegebenenfalls R9 können in den Cyclen bzw. Bicyclen R6 an jeder denkbaren Position angebunden sein. Als beispielhafte substituierte Reste R6 seien genannt: 4-Methylphenyl, 3-Dimethylaminomethylphenyl, 3-Piperidinomethylphenyl, 3-Carboxymethylphenyl, 2-Dimethylaminomethyl-5-methyl-3-furyl, 1-Methylpyrrol-3-yl, 4,5-Dimethyl-oxazol-2-yl, 3,5-Dimethyl-isoxazol-4-yl, 4,5-Dimethyl-thiazol-2-yl, 4-Methyl-5-carboxymethyl-thiazol-2-yl, 1-Methyl-imidazol-2-yl, 1-Methyl-pyrazol-3-yl, 1-(2-Dimethylaminoethyl)-pyrazol-3-yl, 5-Methyl-1,3,4-oxadiazol-2-yl, 1-Methyl-1,2,3-triazol-4-yl, 1-Methyl-1,2,4-triazol-3-yl, 1-(2-Dimethylaminoethyl)-1,2,3-triazol-4-yl, 1-Methyl-tetrazol-5-yl, 1-(2-Dimethylaminoethyl)-tetrazol-5-yl, 1-Carboxymethyl-tetrazol-5-yl, 5-Methyl-1,3,4-thiadiazol-2-yl, 5-Trifluormethyl-1,3,4-thiadiazol-2-yl, 1-(2-Hydroxyethyl)-tetrazol-5-yl, 2-Amino-1,3,4-thiadiazol-2-yl, 3-Amino-1,2,4-triazol-5-yl, 4-Methyl-5-trifluormethyl-1,2,4-triazol-3-yl und 4-Amino-pyrimidin-2-yl.

Als Reste -CₘH₂ₘ-_{,} die durch R6-S(O)_{q}- substituiert sind, kommen geradkettige oder verzweigte Reste infrage. Beispielsweise seien genannt der Heptyl-, Isoheptyl- (2-Methylhexyl-), Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl- und der Ethylrest. Als beispielhafte Reste R6-S(O)_{q}-CₘH₂ₘ seien genannt: Phenylthiopentyl, Phenylthioethyl, Phenylthiopropyl, Phenylthiobutyl, 4-Methyl-phenylthioethyl, 4-Methylphenylthiopropyl, 3-Dimethylaminomethyl-phenyl-thioethyl, 3-Dimethylaminomethyl-phenyl-thiopropyl,
3-Piperidinomethyl-phenylthioethyl,
3-Piperidinomethyl-phenylthiopropyl,
3-Piperidinomethyl -phenylthiobutyl,
1-Methylpyrrol-3-thioethyl,
4,5-Dimethyloxazol-2-thiopropyl,
3,5-Dimethyl-isoxazol-5-thioethyl,
3,5-Dimethyl-isoxazol-5-thiopropyl,
Thiazol-2-thioethyl,
Thiazol-2-thiopropyl,
Thiazol-2-thiobutyl,
4-Methyl-5-carboxymethyl-thiazol-2-thiopropyl,
1-Methylimidazol-2-thioethyl,
1-Methylimidazol-2-thiopropyl,
1-Methylimidazol-2-thiobutyl,
Imidazol-2-thioethyl,
Imidazol-2-thiopropyl,
Pyrazol-3-thiopropyl,
1-(2-Dimethylaminoethyl)-pyrazol-2-thioethyl,
1,3,4-Oxadiazol-2-thioethyl,
1,3,4-Oxdiazol-2-thiopropyl,
1,2,3-Triazol-4-thioethyl,
1,2,3-Triazol-4-thiopropyl,
1,2,3-Triazol-4-thiobutyl,
1-Methyl-1,2,3-triazol-4-thioethyl,
1-Methyl-1,2,3-triazol-4-thiopropyl,
1,2,4-Triazol-3-thioethyl,
1,2,4-Triazol-3-thiopropyl,
3-Amino-1,2,4-triazol-5-thioethyl,
3-Amino-1,2,4-triazol-5-thiopropyl,
4-Methyl-5-trifluormethyl-1,2,4-triazol-3-thioethyl,
4-Methyl-5-trifluormethyl-1,2,4-triazol-3-thiopropyl,
1-Methyl-1,2,4-triazol-3-thioethyl,
1-Methyl-1,2,4-triazol-3-thiopropyl,
1-Methyl-1,2,4-triazol-3-thiobutyl,
Tetrazol-5-thioethyl,
Tetrazol-5-thiopropyl,
Tetrazol-5-thiobutyl,
1-Methyl-tetrazol-5-thioethyl,
1-Methyl-tetrazol-5-thiopropyl,
1-Methyl-tetrazol-5-thiobutyl,
1-(2-Dimethylaminoethyl)-tetrazol-5-thioethyl,
1-(2-Dimethylaminoethyl)-tetrazol-5-thiopropyl,
1-(2-Hydroxyethyl)-tetrazol-5-thioethyl,
1-(2-Hydroxyethyl)-tetrazol-5-thiopropyl,
1,3,4-Thiadiazol-2-thioethyl,
1,3,4-Thiadiazol-2-thiopropyl,
5-Methyl-1,3,4-thiadiazol-2-thioethyl,
5-Methyl-1,3,4-thiadiazol-2-thiopropyl,
5-Methyl-1,3,4-thiadiazol-2-thiobutyl,
5-Trifluormethyl-1,3,4-thiadiazol-2-thioethyl,
5-Trifluormethyl-1,3,4-thiadiazol-2-thiopropyl,
1,2,3-Thiadiazol-4-thioethyl,
1,2,3-Thiadiazol-4-thiopropyl,
1-Carboxymethyl-tetrazol-5-thioethyl,
1-Carboxymethyl-tetrazol-5-thiopropyl,
2-Pyridyl-thioethyl,
2-Pyridyl -thiopropyl,
2-Pyridyl-thiobutyl,
4-Pyridyl-thioethyl,
4-Pyridyl-thiopropyl,
4-Pyridyl-thiobutyl,
2-Pyrimidin-thioethyl,
2-Pyrimidin-thiopropyl,
2-Pyrimidin-thiobutyl,
4-Amino-pyrimidin-2-thioethyl,
4-Amino-pyrimidin-2-thiopropyl,
2-Benzimidazol-thioethyl,
2-Benzimidazol-thiopropyl,
4-Methyl-thiazol-5-thioethyl,
4-Methyl-thiazol-5-thiopropyl,
4-Methyl-thiazol-5-thiobutyl,
1-Methoxycarbonylmethyltetrazol-5-thioethyl,
1-Methoxycarbonylmethyltetrazol-5-thiopropyl,
1-Methoxycarbonylmethyltetrazol-5-thiobutyl,
5-Nitroimidazol-1-thioethyl,
5-Nitroimidazol-1-thiopropyl,
5-Nitroimidazol-1-thiobutyl,
2-Methyl-5-nitroimidazol-1-thioethyl,
2-Methyl-5-nitroimidazol-1-thiopropyl und
2-Methyl-5-nitroimidazol-1-thiobutyl.

Als Salze kommen für Verbindungen der Formel I, in denen n die Zahl 0 bedeutet, alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Für Verbindungen der Formel I, in denen n die Zahl 1 bedeutet, kommen als Salze auch Salze mit Basen in Betracht. Als Beispiele für basische Salze seien Lithium-, Natrium-, Kalium-, Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Hervorzuhebende Verbindungen der Formel I sind solche, in denen
- R1: Wasserstoff bedeutet,
- R2: Wasserstoff, Halogen oder Methoxy bedeutet,
- R3: Wasserstoff bedeutet,
- R4: Wasserstoff bedeutet,
- R5: Wasserstoff, 1-4C-Alkyl oder 1-4C-Alkoxy bedeutet,
- R6: einen durch R8 und R9 substituierten Cyclus bedeutet, der ausgewählt ist aus der Gruppe bestehend aus Benzol, Isoxazol, Thiazol, Imidazol, Triazol, Tetrazol, Thiadiazol, Pyridin, Pyridin-N-oxid, Pyrimidin und Benzimidazol,
- R7: Wasserstoff oder 1-4C-Alkyl bedeutet,
- R8: Wasserstoff, 1-4C-Alkyl, Hydroxy, Nitro, Guanidino, Carboxy, 1-4C-Alkoxycarbonyl, durch R10 substituiertes 1-4C-Alkyl oder Amino bedeutet,
- R9: Wasserstoff oder 1-4C-Alkyl bedeutet,
- R10: Hydroxy, Carboxy, 1-4C-Alkoxycarbonyl oder -N(R11)R12 bedeutet, wobei
- R11: Wasserstoff, 1-4C-Alkyl oder -CO-R13 und
- R12: Wasserstoff oder 1-4C-Alkyl bedeutet, oder wobei
- R11 und R12: zusammen und unter Einschluß des Stickstoffatoms, an das beide gebunden sind, einen Piperidinorest darstellen,
- R13: Wasserstoff, 1-4C-Alkyl oder 1-4C-Alkoxy bedeutet,
- m: eine Zahl von 2 bis 4 bedeutet,
- n: die Zahl 0 oder 1 bedeutet,
- p: die Zahl 0 bedeutet und
- q: die Zahl 0 bedeutet,
und ihre Salze.

Besonders hervorzuhebende Verbindungen der Formel I sind solche, in denen
- R1: Wasserstoff bedeutet,
- R2: Wasserstoff, Fluor oder Methoxy bedeutet,
- R3: Wasserstoff bedeutet,
- R4: Wasserstoff bedeutet,
- R5: 1-4C-Alkyl oder 1-4C-Alkoxy bedeutet,
- R6: einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin, Pyrimidin und Benzimidazol,
- R7: Wasserstoff bedeutet,
- R8: Wasserstoff, 1-4C-Alkyl, Hydroxy, Nitro, Guanidino, Carboxy, 1-4C-Alkoxycarbonyl oder durch R10 substituiertes Methyl oder Ethyl bedeutet,
- R9: Wasserstoff oder 1-4C-Alkyl bedeutet,
- R10: Hydroxy, Carboxy oder -N(R11)R12 bedeutet, wobei
- R11: 1-4C-Alkyl und
- R12: 1-4C-Alkyl bedeutet, oder wobei
- R11 und R12: zusammen und unter Einschluß des Stickstoffatoms, an das beide gebunden sind, einen Piperidinorest darstellen,
- m: eine Zahl von 2 bis 4 bedeutet,
- n: die Zahl 0 bedeutet,
- p: die Zahl 0 bedeutet und
- q: die Zahl 0 bedeutet,
und ihre Salze.

Bevorzugte Verbindungen der Formel I sind solche, in denen
- R1: Wasserstoff bedeutet,
- R2: Wasserstoff oder Fluor bedeutet,
- R3: Wasserstoff bedeutet,
- R4: Wasserstoff bedeutet,
- R5: 1-4C-Alkyl oder 1-4C-Alkoxy bedeutet,
- R6: einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin, Pyrimidin und Benzimidazol,
- R7: Wasserstoff bedeutet,
- R8: Wasserstoff, Methyl, Nitro, 1-4C-Alkoxycarbonyl oder durch R10 substituiertes Methyl oder Ethyl bedeutet,
- R9: Wasserstoff bedeutet,
- R10: Hydroxy, Carboxy oder -N(R11)R12 bedeutet, wobei
- R11: Methyl und
- R12: Methyl bedeutet, oder wobei
- R11 und R12: zusammen und unter Einschluß des Stickstoffatoms, an das beide gebunden sind, einen Piperidinorest darstellen,
- m: eine Zahl von 2 bis 4 bedeutet,
- n: die Zahl 0 bedeutet,
- p: die Zahl 0 bedeutet und
- q: die Zahl 0 bedeutet,
und ihre Salze.

Besonders bevorzugte Verbindungen der Formel I sind solche, in denen
- R1: Wasserstoff bedeutet,
- R2: Wasserstoff bedeutet,
- R3: Wasserstoff bedeutet,
- R4: Wasserstoff bedeutet,
- R5: 1-4C-Alkyl oder 1-4C-Alkoxy bedeutet,
- R6: einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin und Pyrimidin,
- R7: Wasserstoff bedeutet,
- R8: Wasserstoff, Methyl oder durch R10 substituiertes Methyl oder Ethyl bedeutet,
- R9: Wasserstoff bedeutet,
- R10: Carboxy oder -N(R11)R12 bedeutet, wobei
- R11: Methyl und
- R12: Methyl bedeutet,
- m: eine Zahl von 2 bis 4 bedeutet,
- n: die Zahl 0 bedeutet,
- p: die Zahl 0 bedeutet und
- q: die Zahl 0 bedeutet,
und ihre Salze.

Beispielhafte erfindungsgemäße Verbindungen sind in der folgenden Tabelle 1 aufgeführt, wobei der Substituent R1 in 5- bzw. 6-Position und der Substituent R2 in 6- bzw. 5-Position steht (wegen der Tautomerie im Benzimidazolring sind bei R4 = H die Positionen 5 und 6 nicht unterscheidbar):

**TABELLE 1**

| Verbindungen der Formel I (siehe beigefügtes Formelblatt) mit den folgenden Substituentenbedeutungen: | | | | | | | | | | |
|---|---|---|---|---|---|---|---|---|---|---|
| R1 | R2 | R3 | R4 | R5 | R6 | R7 | m | n | p | q |
| H | H | H | H | H | Phenyl | H | 2 | 0 | 0 | 0 |
| H | H | H | H | H | 3-Dimethylaminomethylphenyl | H | 2 | 0 | 0 | 0 |
| H | H | H | H | H | 3-Piperidinomethylphenyl | H | 2 | 0 | 0 | 0 |
| H | H | H | H | H | 2-Thiazolyl | H | 2 | 0 | 0 | 0 |
| H | H | H | H | H | 3,4-Dimethyl-2-thiazolyl | H | 2 | 0 | 0 | 0 |
| H | H | H | H | H | 2-Imidazolyl | H | 2 | 0 | 0 | 0 |
| H | H | H | H | H | 1-Methyl-2-imidazolyl | H | 2 | 0 | 0 | 0 |
| H | H | H | H | H | 1,2,3-Triazol-4-yl | H | 2 | 0 | 0 | 0 |
| H | H | H | H | H | 1-Methyl-1,2,3-triazol-4-yl | H | 2 | 0 | 0 | 0 |
| H | H | H | H | H | 1,2,4-Triazol-3-yl | H | 2 | 0 | 0 | 0 |
| H | H | H | H | H | 4-Methyl-1,2,4-triazol-3-yl | H | 2 | 0 | 0 | 0 |
| H | H | H | H | H | Tetrazol-5-yl | H | 2 | 0 | 0 | 0 |
| H | H | H | H | H | 1-Methyltetrazol-5-yl | H | 2 | 0 | 0 | 0 |
| H | H | H | H | H | 1-(2-Dimethylaminoethyl)-tetrazol-5-yl | H | 2 | 0 | 0 | 0 |
| H | H | H | H | H | 1-(2-Hydroxyethyl)-tetrazol-5-yl | H | 2 | 0 | 0 | 0 |
| H | H | H | H | H | 1,2,3-Thiadiazol-4-yl | H | 2 | 0 | 0 | 0 |
| H | H | H | H | H | 1,3,4-Thiadiazol-2-yl | H | 2 | 0 | 0 | 0 |
| H | H | H | H | H | 5-Methyl-1,3'4-thiadiazol-2-yl | H | 2 | 0 | 0 | 0 |
| H | H | H | H | H | 2-Pyridinyl | H | 2 | 0 | 0 | 0 |
| H | H | H | H | H | 4-Pyridinyl | H | 2 | 0 | 0 | 0 |
| H | H | H | H | H | 2-Pyrimidinyl | H | 2 | 0 | 0 | 0 |
| H | H | H | H | H | 2-Benzimidazolyl | H | 2 | 0 | 0 | 0 |
| H | H | H | H | H | 2-Furanyl | H | 2 | 0 | 0 | 0 |
| H | H | H | H | H | 2-Thienyl | H | 2 | 0 | 0 | 0 |
| H | H | H | H | H | 5-Chlor-thiophen-2-yl | H | 2 | 0 | 0 | 0 |
| H | H | H | H | H | 5-(2-Dimethylaminomethyl)-furan-2-yl | H | 2 | 0 | 0 | 0 |
| H | H | H | H | H | 5-Methyl-furan-2-yl | H | 2 | 0 | 0 | 0 |
| H | H | H | H | H | 2-Methyl-5-nitro-1-imidazolyl | H | 2 | 0 | 0 | 0 |
| H | H | H | H | H | 5-Nitro-1-imidazolyl | H | 2 | 0 | 0 | 0 |
| | | | | | | | | | | |
| H | OCH₃ | H | H | H | Phenyl | H | 2 | 0 | 0 | 0 |
| H | OCH₃ | H | H | H | 3-Dimethylaminomethylphenyl | H | 2 | 0 | 0 | 0 |
| H | OCH₃ | H | H | H | 3-Piperidinomethylphenyl | H | 2 | 0 | 0 | 0 |
| H | OCH₃ | H | H | H | 2-Thiazolyl | H | 2 | 0 | 0 | 0 |
| H | OCH₃ | H | H | H | 3,4-Dimethyl-2-thiazolyl | H | 2 | 0 | 0 | 0 |
| H | OCH₃ | H | H | H | 2-Imidazolyl | H | 2 | 0 | 0 | 0 |
| H | OCH₃ | H | H | H | 1-Methyl-2-imidazolyl | H | 2 | 0 | 0 | 0 |
| H | OCH₃ | H | H | H | 1,2,3-Triazol-4-yl | H | 2 | 0 | 0 | 0 |
| H | OCH₃ | H | H | H | 1-Methyl-1,2,3-triazol-4-yl | H | 2 | 0 | 0 | 0 |
| H | OCH₃ | H | H | H | 1,2,4-Triazol-3-yl | H | 2 | 0 | 0 | 0 |
| H | OCH₃ | H | H | H | 4-Methyl-1,2,4-triazol-3-yl | H | 2 | 0 | 0 | 0 |
| H | OCH₃ | H | H | H | Tetrazol-5-yl | H | 2 | 0 | 0 | 0 |
| H | OCH₃ | H | H | H | 1-Methyltetrazol-5-yl | H | 2 | 0 | 0 | 0 |
| H | OCH₃ | H | H | H | 1-(2-Dimethylaminoethyl)-tetrazol-5-yl | H | 2 | 0 | 0 | 0 |
| H | OCH₃ | H | H | H | 1-(2-Hydroxyethyl)-tetrazol-5-yl | H | 2 | 0 | 0 | 0 |
| H | OCH₃ | H | H | H | 1,2,3-Thiadiazol-4-yl | H | 2 | 0 | 0 | 0 |
| H | OCH₃ | H | H | H | 1,3,4-Thiadiazol-2-yl | H | 2 | 0 | 0 | 0 |
| H | OCH₃ | H | H | H | 5-Methyl-1,3,4-thiadiazol-2-yl | H | 2 | 0 | 0 | 0 |
| H | OCH₃ | H | H | H | 2-Pyridinyl | H | 2 | 0 | 0 | 0 |
| H | OCH₃ | H | H | H | 4-Pyridinyl | H | 2 | 0 | 0 | 0 |
| H | OCH₃ | H | H | H | 2-Pyrimidinyl | H | 2 | 0 | 0 | 0 |
| H | OCH₃ | H | H | H | 2-Benzimidazolyl | H | 2 | 0 | 0 | 0 |
| H | OCH₃ | H | H | H | 2-Furanyl | H | 2 | 0 | 0 | 0 |
| H | OCH₃ | H | H | H | 2-Thienyl | H | 2 | 0 | 0 | 0 |
| H | OCH₃ | H | H | H | 5-Chlor-thiophen-2-yl | H | 2 | 0 | 0 | 0 |
| H | OCH₃ | H | H | H | 5-(2-Dimethylaminomethyl)-furan-2-yl | H | 2 | 0 | 0 | 0 |
| H | OCH₃ | H | H | H | 5-Methyl-furan-2-yl | H | 2 | 0 | 0 | 0 |
| H | OCH₃ | H | H | H | 2-Methyl-5-nitro-1-imidazolyl | H | 2 | 0 | 0 | 0 |
| H | OCH₃ | H | H | H | 5-Nitro-1-imidazolyl | H | 2 | 0 | 0 | 0 |
| | | | | | | | | | | |
| H | H | H | H | CH₃ | Phenyl | H | 2 | 0 | 0 | 0 |
| H | H | H | H | CH₃ | 3-Dimethylaminomethylphenyl | H | 2 | 0 | 0 | 0 |
| H | H | H | H | CH₃ | 3-Piperidinomethylphenyl | H | 2 | 0 | 0 | 0 |
| H | H | H | H | CH₃ | 2-Thiazolyl | H | 2 | 0 | 0 | 0 |
| H | H | H | H | CH₃ | 3,4-Dimethyl-2-thiazolyl | H | 2 | 0 | 0 | 0 |
| H | H | H | H | CH₃ | 2-Imidazolyl | H | 2 | 0 | 0 | 0 |
| H | H | H | H | CH₃ | 1-Methyl-2-imidazolyl | H | 2 | 0 | 0 | 0 |
| H | H | H | H | CH₃ | 1,2,3-Triazol-4-yl | H | 2 | 0 | 0 | 0 |
| H | H | H | H | CH₃ | 1-Methyl-1,2,3-triazol-4-yl | H | 2 | 0 | 0 | 0 |
| H | H | H | H | CH₃ | 1,2,4-Triazol-3-yl | H | 2 | 0 | 0 | 0 |
| H | H | H | H | CH₃ | 4-Methyl-1,2,4-triazol-3-yl | H | 2 | 0 | 0 | 0 |
| H | H | H | H | CH₃ | Tetrazol-5-yl | H | 2 | 0 | 0 | 0 |
| H | H | H | H | CH₃ | 1-Methyltetrazol-5-yl | H | 2 | 0 | 0 | 0 |
| H | H | H | H | CH₃ | 1-(2-Dimethylaminoethyl)-tetrazol-5-yl | H | 2 | 0 | 0 | 0 |
| H | H | H | H | CH₃ | 1-(2-Hydroxyethyl)-tetrazol-5-yl | H | 2 | 0 | 0 | 0 |
| H | H | H | H | CH₃ | 1,2,3-Thiadiazol-4-yl | H | 2 | 0 | 0 | 0 |
| H | H | H | H | CH₃ | 1,3,4-Thiadiazol-2-yl | H | 2 | 0 | 0 | 0 |
| H | H | H | H | CH₃ | 5-Methyl-1,3,4-thiadiazol-2-yl | H | 2 | 0 | 0 | 0 |
| H | H | H | H | CH₃ | 2-Pyridinyl | H | 2 | 0 | 0 | 0 |
| H | H | H | H | CH₃ | 4-Pyridinyl | H | 2 | 0 | 0 | 0 |
| H | H | H | H | CH₃ | 2-Pyrimidinyl | H | 2 | 0 | 0 | 0 |
| H | H | H | H | CH₃ | 2-Benzimidazolyl | H | 2 | 0 | 0 | 0 |
| H | H | H | H | CH₃ | 2-Furanyl | H | 2 | 0 | 0 | 0 |
| H | H | H | H | CH₃ | 2-Thienyl | H | 2 | 0 | 0 | 0 |
| H | H | H | H | CH₃ | 5-Chlor-thiophen-2-yl | H | 2 | 0 | 0 | 0 |
| H | H | H | H | CH₃ | 5-(2-Dimethylaminomethyl)-furan-2-yl | H | 2 | 0 | 0 | 0 |
| H | H | H | H | CH₃ | 5-Methyl-furan-2-yl | H | 2 | 0 | 0 | 0 |
| H | H | H | H | CH₃ | 2-Methyl-5-nitro-1-imidazolyl | H | 2 | 0 | 0 | 0 |
| H | H | H | H | CH₃ | 5-Nitro-1-imidazolyl | H | 2 | 0 | 0 | 0 |
| | | | | | | | | | | |
| H | F | H | H | CH₃ | Phenyl | H | 2 | 0 | 0 | 0 |
| H | F | H | H | CH₃ | 3-Dimethylaminomethylphenyl | H | 2 | 0 | 0 | 0 |
| H | F | H | H | CH₃ | 3-Piperidinomethylphenyl | H | 2 | 0 | 0 | 0 |
| H | F | H | H | CH₃ | 2-Thiazolyl | H | 2 | 0 | 0 | 0 |
| H | F | H | H | CH₃ | 3,4-Dimethyl-2-thiazolyl | H | 2 | 0 | 0 | 0 |
| H | F | H | H | CH₃ | 2-Imidazolyl | H | 2 | 0 | 0 | 0 |
| H | F | H | H | CH₃ | 1-Methyl-2-imidazolyl | H | 2 | 0 | 0 | 0 |
| H | F | H | H | CH₃ | 1,2,3-Triazol-4-yl | H | 2 | 0 | 0 | 0 |
| H | F | H | H | CH₃ | 1-Methyl-1,2,3-triazol-4-yl | H | 2 | 0 | 0 | 0 |
| H | F | H | H | CH₃ | 1,2,4-Triazol-3-yl | H | 2 | 0 | 0 | 0 |
| H | F | H | H | CH₃ | 4-Methyl-1,2,4-triazol-3-yl | H | 2 | 0 | 0 | 0 |
| H | F | H | H | CH₃ | Tetrazol-5-yl | H | 2 | 0 | 0 | 0 |
| H | F | H | H | CH₃ | 1-Methyltetrazol-5-yl | H | 2 | 0 | 0 | 0 |
| H | F | H | H | CH₃ | 1-(2-Dimethylaminoethyl)-tetrazol-5-yl | H | 2 | 0 | 0 | 0 |
| H | F | H | H | CH₃ | 1-(2-Hydroxyethyl)-tetrazol-5-yl | H | 2 | 0 | 0 | 0 |
| H | F | H | H | CH₃ | 1,2,3-Thiadiazol-4-yl | H | 2 | 0 | 0 | 0 |
| H | F | H | H | CH₃ | 1,3,4-Thiadiazol-2-yl | H | 2 | 0 | 0 | 0 |
| H | F | H | H | CH₃ | 5-Methyl-1,3,4-thiadiazol-2-yl | H | 2 | 0 | 0 | 0 |
| H | F | H | H | CH₃ | 2-Pyridinyl | H | 2 | 0 | 0 | 0 |
| H | F | H | H | CH₃ | 4-Pyridinyl | H | 2 | 0 | 0 | 0 |
| H | F | H | H | CH₃ | 2-Pyrimidinyl | H | 2 | 0 | 0 | 0 |
| H | F | H | H | CH₃ | 2-Benzimidazolyl | H | 2 | 0 | 0 | 0 |
| H | F | H | H | H | 2-Furanyl | H | 2 | 0 | 0 | 0 |
| H | F | H | H | H | 2-Thienyl | H | 2 | 0 | 0 | 0 |
| H | F | H | H | H | 5-Chlor-thiophen-2-yl | H | 2 | 0 | 0 | 0 |
| H | F | H | H | H | 5-(2-Dimethylaminomethyl)-furan-2-yl | H | 2 | 0 | 0 | 0 |
| H | F | H | H | H | 5-Methyl-furan-2-yl | H | 2 | 0 | 0 | 0 |
| H | F | H | H | H | 2-Methyl-5-nitro-1-imidazolyl | H | 2 | 0 | 0 | 0 |
| H | F | H | H | H | 5-Nitro-1-imidazolyl | H | 2 | 0 | 0 | 0 |
| | | | | | | | | | | |
| H | H | H | H | OCH₃ | Phenyl | H | 2 | 0 | 0 | 0 |
| H | H | H | H | OCH₃ | 3-Dimethylaminomethylphenyl | H | 2 | 0 | 0 | 0 |
| H | H | H | H | OCH₃ | 3-Piperidinomethylphenyl | H | 2 | 0 | 0 | 0 |
| H | H | H | H | OCH₃ | 2-Thiazolyl | H | 2 | 0 | 0 | 0 |
| H | H | H | H | OCH₃ | 3,4-Dimethyl-2-thiazolyl | H | 2 | 0 | 0 | 0 |
| H | H | H | H | OCH₃ | 2-Imidazolyl | H | 2 | 0 | 0 | 0 |
| H | H | H | H | OCH₃ | 1-Methyl-2-imidazolyl | H | 2 | 0 | 0 | 0 |
| H | H | H | H | OCH₃ | 1,2,3-Triazol-4-yl | H | 2 | 0 | 0 | 0 |
| H | H | H | H | OCH₃ | 1-Methyl-1,2,3-triazol-4-yl | H | 2 | 0 | 0 | 0 |
| H | H | H | H | OCH₃ | 1,2,4-Triazol-3-yl | H | 2 | 0 | 0 | 0 |
| H | H | H | H | OCH₃ | 4-Methyl-1,2,4-triazol-3-yl | H | 2 | 0 | 0 | 0 |
| H | H | H | H | OCH₃ | Tetrazol-5-yl | H | 2 | 0 | 0 | 0 |
| H | H | H | H | OCH₃ | 1-Methyltetrazol-5-yl | H | 2 | 0 | 0 | 0 |
| H | H | H | H | OCH₃ | 1-(2-Dimethylaminoethyl)-tetrazol-5-yl | H | 2 | 0 | 0 | 0 |
| H | H | H | H | OCH₃ | 1-(2-Hydroxyethyl)-tetrazol-5-yl | H | 2 | 0 | 0 | 0 |
| H | H | H | H | OCH₃ | 1,2,3-Thiadiazol-4-yl | H | 2 | 0 | 0 | 0 |
| H | H | H | H | OCH₃ | 1,3,4-Thiadiazol-2-yl | H | 2 | 0 | 0 | 0 |
| H | H | H | H | OCH₃ | 5-Methyl-1,3,4-thiadiazol-2-yl | H | 2 | 0 | 0 | 0 |
| H | H | H | H | OCH₃ | 2-Pyridinyl | H | 2 | 0 | 0 | 0 |
| H | H | H | H | OCH₃ | 4-Pyridinyl | H | 2 | 0 | 0 | 0 |
| H | H | H | H | OCH₃ | 2-Pyrimidinyl | H | 2 | 0 | 0 | 0 |
| H | H | H | H | OCH₃ | 2-Benzimidazolyl | H | 2 | 0 | 0 | 0 |
| H | H | H | H | OCH₃ | 2-Furanyl | H | 2 | 0 | 0 | 0 |
| H | H | H | H | OCH₃ | 2-Thienyl | H | 2 | 0 | 0 | 0 |
| H | H | H | H | OCH₃ | 5-Chlor-thiophen-2-yl | H | 2 | 0 | 0 | 0 |
| H | H | H | H | OCH₃ | 5-(2-Dimethylaminomethyl)-furan-2-yl | H | 2 | 0 | 0 | 0 |
| H | H | H | H | OCH₃ | 5-Methyl-furan-2-yl | H | 2 | 0 | 0 | 0 |
| H | H | H | H | OCH₃ | 2-Methyl-5-nitro-1-imidazolyl | H | 2 | 0 | 0 | 0 |
| H | H | H | H | OCH₃ | 5-Nitro-1-imidazolyl | H | 2 | 0 | 0 | 0 |
| | | | | | | | | | | |
| H | H | H | H | H | Phenyl | H | 3 | 0 | 0 | 0 |
| H | H | H | H | H | 3-Dimethylaminomethylphenyl | H | 3 | 0 | 0 | 0 |
| H | H | H | H | H | 3-Piperidinomethylphenyl | H | 3 | 0 | 0 | 0 |
| H | H | H | H | H | 2-Thiazolyl | H | 3 | 0 | 0 | 0 |
| H | H | H | H | H | 3,4-Dimethyl-2-thiazolyl | H | 3 | 0 | 0 | 0 |
| H | H | H | H | H | 2-Imidazolyl | H | 3 | 0 | 0 | 0 |
| H | H | H | H | H | 1-Methyl-2-imidazolyl | H | 3 | 0 | 0 | 0 |
| H | H | H | H | H | 1,2,3-Triazol-4-yl | H | 3 | 0 | 0 | 0 |
| H | H | H | H | H | 1-Methyl-1,2,3-triazol-4-yl | H | 3 | 0 | 0 | 0 |
| H | H | H | H | H | 1,2,4-Triazol-3-yl | H | 3 | 0 | 0 | 0 |
| H | H | H | H | H | 4-Methyl-1,2,4-triazol-3-yl | H | 3 | 0 | 0 | 0 |
| H | H | H | H | H | Tetrazol-5-yl | H | 3 | 0 | 0 | 0 |
| H | H | H | H | H | 1-Methyltetrazol-5-yl | H | 3 | 0 | 0 | 0 |
| H | H | H | H | H | 1-(2-Dimethylaminoethyl)-tetrazol-5-yl | H | 3 | 0 | 0 | 0 |
| H | H | H | H | H | 1-(2-Hydroxyethyl)-tetrazol-5-yl | H | 3 | 0 | 0 | 0 |
| H | H | H | H | H | 1,2,3-Thiadiazol-4-yl | H | 3 | 0 | 0 | 0 |
| H | H | H | H | H | 1,3,4-Thiadiazol-2-yl | H | 3 | 0 | 0 | 0 |
| H | H | H | H | H | 5-Methyl-1,3,4-thiadiazol-2-yl | H | 3 | 0 | 0 | 0 |
| H | H | H | H | H | 2-Pyridinyl | H | 3 | 0 | 0 | 0 |
| H | H | H | H | H | 4-Pyridinyl | H | 3 | 0 | 0 | 0 |
| H | H | H | H | H | 2-Pyrimidinyl | H | 3 | 0 | 0 | 0 |
| H | H | H | H | H | 2-Benzimidazolyl | H | 3 | 0 | 0 | 0 |
| H | H | H | H | H | 2-Furanyl | H | 3 | 0 | 0 | 0 |
| H | H | H | H | H | 2-Thienyl | H | 3 | 0 | 0 | 0 |
| H | H | H | H | H | 5-Chlor-thiophen-2-yl | H | 3 | 0 | 0 | 0 |
| H | H | H | H | H | 5-(2-Dimethylaminomethyl)-furan-2-yl | H | 3 | 0 | 0 | 0 |
| H | H | H | H | H | 5-Methyl-furan-2-yl | H | 3 | 0 | 0 | 0 |
| H | H | H | H | H | 2-Methyl-5-nitro-1-imidazolyl | H | 3 | 0 | 0 | 0 |
| H | H | H | H | H | 5-Nitro-1-imidazolyl | H | 3 | 0 | 0 | 0 |
| H | OCH₃ | H | H | H | Phenyl | H | 3 | 0 | 0 | 0 |
| H | OCH₃ | H | H | H | 3-Dimethylaminomethylphenyl | H | 3 | 0 | 0 | 0 |
| H | OCH₃ | H | H | H | 3-Piperidinomethylphenyl | H | 3 | 0 | 0 | 0 |
| H | OCH₃ | H | H | H | 2-Thiazolyl | H | 3 | 0 | 0 | 0 |
| H | OCH₃ | H | H | H | 3,4-Dimethyl-2-thiazolyl | H | 3 | 0 | 0 | 0 |
| H | OCH₃ | H | H | H | 2-Imidazolyl | H | 3 | 0 | 0 | 0 |
| H | OCH₃ | H | H | H | 1-Methyl-2-imidazolyl | H | 3 | 0 | 0 | 0 |
| H | OCH₃ | H | H | H | 1,2,3-Triazol-4-yl | H | 3 | 0 | 0 | 0 |
| H | OCH₃ | H | H | H | 1-Methyl-1,2,3-triazol-4-yl | H | 3 | 0 | 0 | 0 |
| H | OCH₃ | H | H | H | 1,2,4-Triazol-3-yl | H | 3 | 0 | 0 | 0 |
| H | OCH₃ | H | H | H | 4-Methyl-1,2,4-triazol-3-yl | H | 3 | 0 | 0 | 0 |
| H | OCH₃ | H | H | H | Tetrazol-5-yl | H | 3 | 0 | 0 | 0 |
| H | OCH₃ | H | H | H | 1-Methyltetrazol-5-yl | H | 3 | 0 | 0 | 0 |
| H | OCH₃ | H | H | H | 1-(2-Dimethylaminoethyl)-tetrazol-5-yl | H | 3 | 0 | 0 | 0 |
| H | OCH₃ | H | H | H | 1-(2-Hydroxyethyl)-tetrazol-5-yl | H | 3 | 0 | 0 | 0 |
| H | OCH₃ | H | H | H | 1,2,3-Thiadiazol-4-yl | H | 3 | 0 | 0 | 0 |
| H | OCH₃ | H | H | H | 1,3,4-Thiadiazol-2-yl | H | 3 | 0 | 0 | 0 |
| H | OCH₃ | H | H | H | 5-Methyl-1,3,4-thiadiazol-2-yl | H | 3 | 0 | 0 | 0 |
| H | OCH₃ | H | H | H | 2-Pyridinyl | H | 3 | 0 | 0 | 0 |
| H | OCH₃ | H | H | H | 4-Pyridinyl | H | 3 | 0 | 0 | 0 |
| H | OCH₃ | H | H | H | 2-Pyrimidinyl | H | 3 | 0 | 0 | 0 |
| H | OCH₃ | H | H | H | 2-Benzimidazolyl | H | 3 | 0 | 0 | 0 |
| H | OCH₃ | H | H | H | 2-Furanyl | H | 3 | 0 | 0 | 0 |
| H | OCH₃ | H | H | H | 2-Thienyl | H | 3 | 0 | 0 | 0 |
| H | OCH₃ | H | H | H | 5-Chlor-thiophen-2-yl | H | 3 | 0 | 0 | 0 |
| H | OCH₃ | H | H | H | 5-(2-Dimethylaminomethyl)-furan-2-yl | H | 3 | 0 | 0 | 0 |
| H | OCH₃ | H | H | H | 5-Methyl-furan-2-yl | H | 3 | 0 | 0 | 0 |
| H | OCH₃ | H | H | H | 2-Methyl-5-nitro-1-imidazolyl | H | 3 | 0 | 0 | 0 |
| H | OCH₃ | H | H | H | 5-Nitro-1-imidazolyl | H | 3 | 0 | 0 | 0 |
| | | | | | | | | | | |
| H | H | H | H | CH₃ | Phenyl | H | 3 | 0 | 0 | 0 |
| H | H | H | H | CH₃ | 3-Dimethylaminomethylphenyl | H | 3 | 0 | 0 | 0 |
| H | H | H | H | CH₃ | 3-Piperidinomethylphenyl | H | 3 | 0 | 0 | 0 |
| H | H | H | H | CH₃ | 2-Thiazolyl | H | 3 | 0 | 0 | 0 |
| H | H | H | H | CH₃ | 3,4-Dimethyl-2-thiazolyl | H | 3 | 0 | 0 | 0 |
| H | H | H | H | CH₃ | 2-Imidazolyl | H | 3 | 0 | 0 | 0 |
| H | H | H | H | CH₃ | 1-Methyl-2-imidazolyl | H | 3 | 0 | 0 | 0 |
| H | H | H | H | CH₃ | 1,2,3-Triazol-4-yl | H | 3 | 0 | 0 | 0 |
| H | H | H | H | CH₃ | 1-Methyl-1,2,3-triazol-4-yl | H | 3 | 0 | 0 | 0 |
| H | H | H | H | CH₃ | 1,2,4-Triazol-3-yl | H | 3 | 0 | 0 | 0 |
| H | H | H | H | CH₃ | 4-Methyl-1,2,4-triazol-3-yl | H | 3 | 0 | 0 | 0 |
| H | H | H | H | CH₃ | Tetrazol-5-yl | H | 3 | 0 | 0 | 0 |
| H | H | H | H | CH₃ | 1-Methyltetrazol-5-yl | H | 3 | 0 | 0 | 0 |
| H | H | H | H | CH₃ | 1-(2-Dimethylaminoethyl)-tetrazol-5-yl | H | 3 | 0 | 0 | 0 |
| H | H | H | H | CH₃ | 1-(2-Hydroxyethyl)-tetrazol-5-yl | H | 3 | 0 | 0 | 0 |
| H | H | H | H | CH₃ | 1,2,3-Thiadiazol-4-yl | H | 3 | 0 | 0 | 0 |
| H | H | H | H | CH₃ | 1,3,4-Thiadiazol-2-yl | H | 3 | 0 | 0 | 0 |
| H | H | H | H | CH₃ | 5-Methyl-1,3,4-thiadiazol-2-yl | H | 3 | 0 | 0 | 0 |
| H | H | H | H | CH₃ | 2-Pyridinyl | H | 3 | 0 | 0 | 0 |
| H | H | H | H | CH₃ | 4-Pyridinyl | H | 3 | 0 | 0 | 0 |
| H | H | H | H | CH₃ | 2-Pyrimidinyl | H | 3 | 0 | 0 | 0 |
| H | H | H | H | CH₃ | 2-Benzimidazolyl | H | 3 | 0 | 0 | 0 |
| H | H | H | H | CH₃ | 2-Furanyl | H | 3 | 0 | 0 | 0 |
| H | H | H | H | CH₃ | 2-Thienyl | H | 3 | 0 | 0 | 0 |
| H | H | H | H | CH₃ | 5-Chlor-thiophen-2-yl | H | 3 | 0 | 0 | 0 |
| H | H | H | H | CH₃ | 5-(2-Dimethylaminomethyl)-furan-2-yl | H | 3 | 0 | 0 | 0 |
| H | H | H | H | CH₃ | 5-Methyl-furan-2-yl | H | 3 | 0 | 0 | 0 |
| H | H | H | H | CH₃ | 2-Methyl-5-nitro-1-imidazolyl | H | 3 | 0 | 0 | 0 |
| H | H | H | H | CH₃ | 5-Nitro-1-imidazolyl | H | 3 | 0 | 0 | 0 |
| | | | | | | | | | | |
| H | F | H | H | CH₃ | Phenyl | H | 3 | 0 | 0 | 0 |
| H | F | H | H | CH₃ | 3-Dimethylaminomethylphenyl | H | 3 | 0 | 0 | 0 |
| H | F | H | H | CH₃ | 3-Piperidinomethylphenyl | H | 3 | 0 | 0 | 0 |
| H | F | H | H | CH₃ | 2-Thiazolyl | H | 3 | 0 | 0 | 0 |
| H | F | H | H | CH₃ | 3,4-Dimethyl-2-thiazolyl | H | 3 | 0 | 0 | 0 |
| H | F | H | H | CH₃ | 2-Imidazolyl | H | 3 | 0 | 0 | 0 |
| H | F | H | H | CH₃ | 1-Methyl-2-imidazolyl | H | 3 | 0 | 0 | 0 |
| H | F | H | H | CH₃ | 1,2,3-Triazol-4-yl | H | 3 | 0 | 0 | 0 |
| H | F | H | H | CH₃ | 1-Methyl-1,2,3-triazol-4-yl | H | 3 | 0 | 0 | 0 |
| H | F | H | H | CH₃ | 1,2,4-Triazol-3-yl | H | 3 | 0 | 0 | 0 |
| H | F | H | H | CH₃ | 4-Methyl-1,2,4-triazol-3-yl | H | 3 | 0 | 0 | 0 |
| H | F | H | H | CH₃ | Tetrazol-5-yl | H | 3 | 0 | 0 | 0 |
| H | F | H | H | CH₃ | 1-Methyltetrazol-5-yl | H | 3 | 0 | 0 | 0 |
| H | F | H | H | CH₃ | 1-(2-Dimethylaminoethyl)-tetrazol-5-yl | H | 3 | 0 | 0 | 0 |
| H | F | H | H | CH₃ | 1-(2-Hydroxyethyl)-tetrazol-5-yl | H | 3 | 0 | 0 | 0 |
| H | F | H | H | CH₃ | 1,2,3-Thiadiazol-4-yl | H | 3 | 0 | 0 | 0 |
| H | F | H | H | CH₃ | 1,3,4-Thiadiazol-2-yl | H | 3 | 0 | 0 | 0 |
| H | F | H | H | CH₃ | 5-Methyl-1,3,4-thiadiazol-2-yl | H | 3 | 0 | 0 | 0 |
| H | F | H | H | CH₃ | 2-Pyridinyl | H | 3 | 0 | 0 | 0 |
| H | F | H | H | CH₃ | 4-Pyridinyl | H | 3 | 0 | 0 | 0 |
| H | F | H | H | CH₃ | 2-Pyrimidinyl | H | 3 | 0 | 0 | 0 |
| H | F | H | H | CH₃ | 2-Benzimidazolyl | H | 3 | 0 | 0 | 0 |
| H | F | H | H | CH₃ | 2-Furanyl | H | 3 | 0 | 0 | 0 |
| H | F | H | H | CH₃ | 2-Thienyl | H | 3 | 0 | 0 | 0 |
| H | F | H | H | CH₃ | 5-Chlor-thiophen-2-yl | H | 3 | 0 | 0 | 0 |
| H | F | H | H | CH₃ | 5-(2-Dimethylaminomethyl)-furan-2-yl | H | 3 | 0 | 0 | 0 |
| H | F | H | H | CH₃ | 5-Methyl-furan-2-yl | H | 3 | 0 | 0 | 0 |
| H | F | H | H | CH₃ | 2-Methyl-5-nitro-1-imidazolyl | H | 3 | 0 | 0 | 0 |
| H | F | H | H | CH₃ | 5-Nitro-1-imidazolyl | H | 3 | 0 | 0 | 0 |
| | | | | | | | | | | |
| H | H | H | H | OCH₃ | Phenyl | H | 3 | 0 | 0 | 0 |
| H | H | H | H | OCH₃ | 3-Dimethylaminomethylphenyl | H | 3 | 0 | 0 | 0 |
| H | H | H | H | OCH₃ | 3-Piperidinomethylphenyl | H | 3 | 0 | 0 | 0 |
| H | H | H | H | OCH₃ | 2-Thiazolyl | H | 3 | 0 | 0 | 0 |
| H | H | H | H | OCH₃ | 3,4-Dimethyl-2-thiazolyl | H | 3 | 0 | 0 | 0 |
| H | H | H | H | OCH₃ | 2-Imidazolyl | H | 3 | 0 | 0 | 0 |
| H | H | H | H | OCH₃ | 1-Methyl-2-imidazolyl | H | 3 | 0 | 0 | 0 |
| H | H | H | H | OCH₃ | 1,2,3-Triazol-4-yl | H | 3 | 0 | 0 | 0 |
| H | H | H | H | OCH₃ | 1-Methyl-1,2,3-triazol-4-yl | H | 3 | 0 | 0 | 0 |
| H | H | H | H | OCH₃ | 1,2,4-Triazol-3-yl | H | 3 | 0 | 0 | 0 |
| H | H | H | H | OCH₃ | 4-Methyl-1,2,4-triazol-3-yl | H | 3 | 0 | 0 | 0 |
| H | H | H | H | OCH₃ | Tetrazol-5-yl | H | 3 | 0 | 0 | 0 |
| H | H | H | H | OCH₃ | 1-Methyltetrazol-5-yl | H | 3 | 0 | 0 | 0 |
| H | H | H | H | OCH₃ | 1-(2-Dimethylaminoethyl)-tetrazol-5-yl | H | 3 | 0 | 0 | 0 |
| H | H | H | H | OCH₃ | 1-(2-Hydroxyethyl)-tetrazol-5-yl | H | 3 | 0 | 0 | 0 |
| H | H | H | H | OCH₃ | 1,2,3-Thiadiazol-4-yl | H | 3 | 0 | 0 | 0 |
| H | H | H | H | OCH₃ | 1,3,4-Thiadiazol-2-yl | H | 3 | 0 | 0 | 0 |
| H | H | H | H | OCH₃ | 5-Methyl-1,3,4-thiadiazol-2-yl | H | 3 | 0 | 0 | 0 |
| H | H | H | H | OCH₃ | 2-Pyridinyl | H | 3 | 0 | 0 | 0 |
| H | H | H | H | OCH₃ | 4-Pyridinyl | H | 3 | 0 | 0 | 0 |
| H | H | H | H | OCH₃ | 2-Pyrimidinyl | H | 3 | 0 | 0 | 0 |
| H | H | H | H | OCH₃ | 2-Benzimidazolyl | H | 3 | 0 | 0 | 0 |
| H | H | H | H | OCH₃ | 2-Furanyl | H | 3 | 0 | 0 | 0 |
| H | H | H | H | OCH₃ | 2-Thienyl | H | 3 | 0 | 0 | 0 |
| H | H | H | H | OCH₃ | 5-Chlor-thiophen-2-yl | H | 3 | 0 | 0 | 0 |
| H | H | H | H | OCH₃ | 5-(2-Dimethylaminomethyl)-furan-2-yl | H | 3 | 0 | 0 | 0 |
| H | H | H | H | OCH₃ | 5-Methyl-furan-2-yl | H | 3 | 0 | 0 | 0 |
| H | H | H | H | OCH₃ | 2-Methyl-5-nitro-1-imidazolyl | H | 3 | 0 | 0 | 0 |
| H | H | H | H | OCH₃ | 5-Nitro-1-imidazolyl | H | 3 | 0 | 0 | 0 |
| | | | | | | | | | | |
| H | H | H | H | H | Phenyl | H | 4 | 0 | 0 | 0 |
| H | H | H | H | H | 3-Dimethylaminomethylphenyl | H | 4 | 0 | 0 | 0 |
| H | H | H | H | H | 3-Piperidinomethylphenyl | H | 4 | 0 | 0 | 0 |
| H | H | H | H | H | 2-Thiazolyl | H | 4 | 0 | 0 | 0 |
| H | H | H | H | H | 3,4-Dimethyl-2-thiazolyl | H | 4 | 0 | 0 | 0 |
| H | H | H | H | H | 2-Imidazolyl | H | 4 | 0 | 0 | 0 |
| H | H | H | H | H | 1-Methyl-2-imidazolyl | H | 4 | 0 | 0 | 0 |
| H | H | H | H | H | 1,2,3-Triazol-4-yl | H | 4 | 0 | 0 | 0 |
| H | H | H | H | H | 1-Methyl-1,2,3-triazol-4-yl | H | 4 | 0 | 0 | 0 |
| H | H | H | H | H | 1,2,4-Triazol-3-yl | H | 4 | 0 | 0 | 0 |
| H | H | H | H | H | 4-Methyl-1,2,4-triazol-3-yl | H | 4 | 0 | 0 | 0 |
| H | H | H | H | H | Tetrazol-5-yl | H | 4 | 0 | 0 | 0 |
| H | H | H | H | H | 1-Methyltetrazol-5-yl | H | 4 | 0 | 0 | 0 |
| H | H | H | H | H | 1-(2-Dimethylaminoethyl)-tetrazol-5-yl | H | 4 | 0 | 0 | 0 |
| H | H | H | H | H | 1-(2-Hydroxyethyl)-tetrazol-5-yl | H | 4 | 0 | 0 | 0 |
| H | H | H | H | H | 1,2,3-Thiadiazol-4-yl | H | 4 | 0 | 0 | 0 |
| H | H | H | H | H | 1,3,4-Thiadiazol-2-yl | H | 4 | 0 | 0 | 0 |
| H | H | H | H | H | 5-Methyl-1,3,4-thiadiazol-2-yl | H | 4 | 0 | 0 | 0 |
| H | H | H | H | H | 2-Pyridinyl | H | 4 | 0 | 0 | 0 |
| H | H | H | H | H | 4-Pyridinyl | H | 4 | 0 | 0 | 0 |
| H | H | H | H | H | 2-Pyrimidinyl | H | 4 | 0 | 0 | 0 |
| H | H | H | H | H | 2-Benzimidazolyl | H | 4 | 0 | 0 | 0 |
| H | H | H | H | H | 2-Furanyl | H | 4 | 0 | 0 | 0 |
| H | H | H | H | H | 2-Thienyl | H | 4 | 0 | 0 | 0 |
| H | H | H | H | H | 5-Chlor-thiophen-2-yl | H | 4 | 0 | 0 | 0 |
| H | H | H | H | H | 5-(2-Dimethylaminomethyl)-furan-2-yl | H | 4 | 0 | 0 | 0 |
| H | H | H | H | H | 5-Methyl-furan-2-yl | H | 4 | 0 | 0 | 0 |
| H | H | H | H | H | 2-Methyl-5-nitro-1-imidazolyl | H | 4 | 0 | 0 | 0 |
| H | H | H | H | H | 5-Nitro-1-imidazolyl | H | 4 | 0 | 0 | 0 |
| | | | | | | | | | | |
| H | OCH₃ | H | H | H | Phenyl | H | 4 | 0 | 0 | 0 |
| H | OCH₃ | H | H | H | 3-Dimethylaminomethylphenyl | H | 4 | 0 | 0 | 0 |
| H | OCH₃ | H | H | H | 3-Piperidinomethylphenyl | H | 4 | 0 | 0 | 0 |
| H | OCH₃ | H | H | H | 2-Thiazolyl | H | 4 | 0 | 0 | 0 |
| H | OCH₃ | H | H | H | 3,4-Dimethyl-2-thiazolyl | H | 4 | 0 | 0 | 0 |
| H | OCH₃ | H | H | H | 2-Imidazolyl | H | 4 | 0 | 0 | 0 |
| H | OCH₃ | H | H | H | 1-Methyl-2-imidazolyl | H | 4 | 0 | 0 | 0 |
| H | OCH₃ | H | H | H | 1,2,3-Triazol-4-yl | H | 4 | 0 | 0 | 0 |
| H | OCH₃ | H | H | H | 1-Methyl-1,2,3-triazol-4-yl | H | 4 | 0 | 0 | 0 |
| H | OCH₃ | H | H | H | 1,2,4-Triazol-3-yl | H | 4 | 0 | 0 | 0 |
| H | OCH₃ | H | H | H | 4-Methyl-1,2,4-triazol-3-yl | H | 4 | 0 | 0 | 0 |
| H | OCH₃ | H | H | H | Tetrazol-5-yl | H | 4 | 0 | 0 | 0 |
| H | OCH₃ | H | H | H | 1-Methyltetrazol-5-yl | H | 4 | 0 | 0 | 0 |
| H | OCH₃ | H | H | H | 1-(2-Dimethylaminoethyl)-tetrazol-5-yl | H | 4 | 0 | 0 | 0 |
| H | OCH₃ | H | H | H | 1-(2-Hydroxyethyl)-tetrazol-5-yl | H | 4 | 0 | 0 | 0 |
| H | OCH₃ | H | H | H | 1,2,3-Thiadiazol-4-yl | H | 4 | 0 | 0 | 0 |
| H | OCH₃ | H | H | H | 1,3,4-Thiadiazol-2-yl | H | 4 | 0 | 0 | 0 |
| H | OCH₃ | H | H | H | 5-Methyl-1,3,4-thiadiazol-2-yl | H | 4 | 0 | 0 | 0 |
| H | OCH₃ | H | H | H | 2-Pyridinyl | H | 4 | 0 | 0 | 0 |
| H | OCH₃ | H | H | H | 4-Pyridinyl | H | 4 | 0 | 0 | 0 |
| H | OCH₃ | H | H | H | 2-Pyrimidinyl | H | 4 | 0 | 0 | 0 |
| H | OCH₃ | H | H | H | 2-Benzimidazolyl | H | 4 | 0 | 0 | 0 |
| H | OCH₃ | H | H | H | 2-Furanyl | H | 4 | 0 | 0 | 0 |
| H | OCH₃ | H | H | H | 2-Thienyl | H | 4 | 0 | 0 | 0 |
| H | OCH₃ | H | H | H | 5-Chlor-thiophen-2-yl | H | 4 | 0 | 0 | 0 |
| H | OCH₃ | H | H | H | 5-(2-Dimethylaminomethyl)-furan-2-yl | H | 4 | 0 | 0 | 0 |
| H | OCH₃ | H | H | H | 5-Methyl-furan-2-yl | H | 4 | 0 | 0 | 0 |
| H | OCH₃ | H | H | H | 2-Methyl-5-nitro-1-imidazolyl | H | 4 | 0 | 0 | 0 |
| H | OCH₃ | H | H | H | 5-Nitro-1-imidazolyl | H | 4 | 0 | 0 | 0 |
| | | | | | | | | | | |
| H | H | H | H | CH₃ | Phenyl | H | 4 | 0 | 0 | 0 |
| H | H | H | H | CH₃ | 3-Dimethylaminomethylphenyl | H | 4 | 0 | 0 | 0 |
| H | H | H | H | CH₃ | 3-Piperidinomethylphenyl | H | 4 | 0 | 0 | 0 |
| H | H | H | H | CH₃ | 2-Thiazolyl | H | 4 | 0 | 0 | 0 |
| H | H | H | H | CH₃ | 3,4-Dimethyl-2-thiazolyl | H | 4 | 0 | 0 | 0 |
| H | H | H | H | CH₃ | 2-Imidazolyl | H | 4 | 0 | 0 | 0 |
| H | H | H | H | CH₃ | 1-Methyl-2-imidazolyl | H | 4 | 0 | 0 | 0 |
| H | H | H | H | CH₃ | 1,2,3-Triazol-4-yl | H | 4 | 0 | 0 | 0 |
| H | H | H | H | CH₃ | 1-Methyl-1,2,3-triazol-4-yl | H | 4 | 0 | 0 | 0 |
| H | H | H | H | CH₃ | 1,2,4-Triazol-3-yl | H | 4 | 0 | 0 | 0 |
| H | H | H | H | CH₃ | 4-Methyl-1,2,4-triazol-3-yl | H | 4 | 0 | 0 | 0 |
| H | H | H | H | CH₃ | Tetrazol-5-yl | H | 4 | 0 | 0 | 0 |
| H | H | H | H | CH₃ | 1-Methyltetrazol-5-yl | H | 4 | 0 | 0 | 0 |
| H | H | H | H | CH₃ | 1-(2-Dimethylaminoethyl)-tetrazol-5-yl | H | 4 | 0 | 0 | 0 |
| H | H | H | H | CH₃ | 1-(2-Hydroxyethyl)-tetrazol-5-yl | H | 4 | 0 | 0 | 0 |
| H | H | H | H | CH₃ | 1,2,3-Thiadiazol-4-yl | H | 4 | 0 | 0 | 0 |
| H | H | H | H | CH₃ | 1,3,4-Thiadiazol-2-yl | H | 4 | 0 | 0 | 0 |
| H | H | H | H | CH₃ | 5-Methyl-1,3,4-thiadiazol-2-yl | H | 4 | 0 | 0 | 0 |
| H | H | H | H | CH₃ | 2-Pyridinyl | H | 4 | 0 | 0 | 0 |
| H | H | H | H | CH₃ | 4-Pyridinyl | H | 4 | 0 | 0 | 0 |
| H | H | H | H | CH₃ | 2-Pyrimidinyl | H | 4 | 0 | 0 | 0 |
| H | H | H | H | CH₃ | 2-Benzimidazolyl | H | 4 | 0 | 0 | 0 |
| H | H | H | H | CH₃ | 2-Furanyl | H | 4 | 0 | 0 | 0 |
| H | H | H | H | CH₃ | 2-Thienyl | H | 4 | 0 | 0 | 0 |
| H | H | H | H | CH₃ | 5-Chlor-thiophen-2-yl | H | 4 | 0 | 0 | 0 |
| H | H | H | H | CH₃ | 5-(2-Dimethylaminomethyl)-furan-2-yl | H | 4 | 0 | 0 | 0 |
| H | H | H | H | CH₃ | 5-Methyl-furan-2-yl | H | 4 | 0 | 0 | 0 |
| H | H | H | H | CH₃ | 2-Methyl-5-nitro-1-imidazolyl | H | 4 | 0 | 0 | 0 |
| H | H | H | H | CH₃ | 5-Nitro-1-imidazolyl | H | 4 | 0 | 0 | 0 |
| | | | | | | | | | | |
| H | F | H | H | CH₃ | Phenyl | H | 4 | 0 | 0 | 0 |
| H | F | H | H | CH₃ | 3-Dimethylaminomethylphenyl | H | 4 | 0 | 0 | 0 |
| H | F | H | H | CH₃ | 3-Piperidinomethylphenyl | H | 4 | 0 | 0 | 0 |
| H | F | H | H | CH₃ | 2-Thiazolyl | H | 4 | 0 | 0 | 0 |
| H | F | H | H | CH₃ | 3,4-Dimethyl-2-thiazolyl | H | 4 | 0 | 0 | 0 |
| H | F | H | H | CH₃ | 2-Imidazolyl | H | 4 | 0 | 0 | 0 |
| H | F | H | H | CH₃ | 1-Methyl-2-imidazolyl | H | 4 | 0 | 0 | 0 |
| H | F | H | H | CH₃ | 1,2,3-Triazol-4-yl | H | 4 | 0 | 0 | 0 |
| H | F | H | H | CH₃ | 1-Methyl-1,2,3-triazol-4-yl | H | 4 | 0 | 0 | 0 |
| H | F | H | H | CH₃ | 1,2,4-Triazol-3-yl | H | 4 | 0 | 0 | 0 |
| H | F | H | H | CH₃ | 4-Methyl-1,2,4-triazol-3-yl | H | 4 | 0 | 0 | 0 |
| H | F | H | H | CH₃ | Tetrazol-5-yl | H | 4 | 0 | 0 | 0 |
| H | F | H | H | CH₃ | 1-Methyltetrazol-5-yl | H | 4 | 0 | 0 | 0 |
| H | F | H | H | CH₃ | 1-(2-Dimethylaminoethyl)-tetrazol-5-yl | H | 4 | 0 | 0 | 0 |
| H | F | H | H | CH₃ | 1-(2-Hydroxyethyl)-tetrazol-5-yl | H | 4 | 0 | 0 | 0 |
| H | F | H | H | CH₃ | 1,2,3-Thiadiazol-4-yl | H | 4 | 0 | 0 | 0 |
| H | F | H | H | CH₃ | 1,3,4-Thiadiazol-2-yl | H | 4 | 0 | 0 | 0 |
| H | F | H | H | CH₃ | 5-Methyl-1,3,4-thiadiazol-2-yl | H | 4 | 0 | 0 | 0 |
| H | F | H | H | CH₃ | 2-Pyridinyl | H | 4 | 0 | 0 | 0 |
| H | F | H | H | CH₃ | 4-Pyridinyl | H | 4 | 0 | 0 | 0 |
| H | F | H | H | CH₃ | 2-Pyrimidinyl | H | 4 | 0 | 0 | 0 |
| H | F | H | H | CH₃ | 2-Benzimidazolyl | H | 4 | 0 | 0 | 0 |
| H | F | H | H | CH₃ | 2-Furanyl | H | 4 | 0 | 0 | 0 |
| H | F | H | H | CH₃ | 2-Thienyl | H | 4 | 0 | 0 | 0 |
| H | F | H | H | CH₃ | 5-Chlor-thiophen-2-yl | H | 4 | 0 | 0 | 0 |
| H | F | H | H | CH₃ | 5-(2-Dimethylaminomethyl)-furan-2-yl | H | 4 | 0 | 0 | 0 |
| H | F | H | H | CH₃ | 5-Methyl-furan-2-yl | H | 4 | 0 | 0 | 0 |
| H | F | H | H | CH₃ | 2-Methyl-5-nitro-1-imidazolyl | H | 4 | 0 | 0 | 0 |
| H | F | H | H | CH₃ | 5-Nitro-1-imidazolyl | H | 4 | 0 | 0 | 0 |
| | | | | | | | | | | |
| H | H | H | H | OCH₃ | Phenyl | H | 4 | 0 | 0 | 0 |
| H | H | H | H | OCH₃ | 3-Dimethylaminomethylphenyl | H | 4 | 0 | 0 | 0 |
| H | H | H | H | OCH₃ | 3-Piperidinomethylphenyl | H | 4 | 0 | 0 | 0 |
| H | H | H | H | OCH₃ | 2-Thiazolyl | H | 4 | 0 | 0 | 0 |
| H | H | H | H | OCH₃ | 3,4-Dimethyl-2-thiazolyl | H | 4 | 0 | 0 | 0 |
| H | H | H | H | OCH₃ | 2-Imidazolyl | H | 4 | 0 | 0 | 0 |
| H | H | H | H | OCH₃ | 1-Methyl-2-imidazolyl | H | 4 | 0 | 0 | 0 |
| H | H | H | H | OCH₃ | 1,2,3-Triazol-4-yl | H | 4 | 0 | 0 | 0 |
| H | H | H | H | OCH₃ | 1-Methyl-1,2,3-triazol-4-yl | H | 4 | 0 | 0 | 0 |
| H | H | H | H | OCH₃ | 1,2,4-Triazol-3-yl | H | 4 | 0 | 0 | 0 |
| H | H | H | H | OCH₃ | 4-Methyl-1,2,4-triazol-3-yl | H | 4 | 0 | 0 | 0 |
| H | H | H | H | OCH₃ | Tetrazol-5-yl | H | 4 | 0 | 0 | 0 |
| H | H | H | H | OCH₃ | 1-Methyltetrazol-5-yl | H | 4 | 0 | 0 | 0 |
| H | H | H | H | OCH₃ | 1-(2-Dimethylaminoethyl)-tetrazol-5-yl | H | 4 | 0 | 0 | 0 |
| H | H | H | H | OCH₃ | 1-(2-Hydroxyethyl)-tetrazol-5-yl | H | 4 | 0 | 0 | 0 |
| H | H | H | H | OCH₃ | 1,2,3-Thiadiazol-4-yl | H | 4 | 0 | 0 | 0 |
| H | H | H | H | OCH₃ | 1,3,4-Thiadiazol-2-yl | H | 4 | 0 | 0 | 0 |
| H | H | H | H | OCH₃ | 5-Methyl-1,3,4-thiadiazol-2-yl | H | 4 | 0 | 0 | 0 |
| H | H | H | H | OCH₃ | 2-Pyridinyl | H | 4 | 0 | 0 | 0 |
| H | H | H | H | OCH₃ | 4-Pyridinyl | H | 4 | 0 | 0 | 0 |
| H | H | H | H | OCH₃ | 2-Pyrimidinyl | H | 4 | 0 | 0 | 0 |
| H | H | H | H | OCH₃ | 2-Benzimidazolyl | H | 4 | 0 | 0 | 0 |
| H | H | H | H | OCH₃ | 2-Furanyl | H | 4 | 0 | 0 | 0 |
| H | H | H | H | OCH₃ | 2-Thienyl | H | 4 | 0 | 0 | 0 |
| H | H | H | H | OCH₃ | 5-Chlor-thiophen-2-yl | H | 4 | 0 | 0 | 0 |
| H | H | H | H | OCH₃ | 5-(2-Dimethylaminomethyl)-furan-2-yl | H | 4 | 0 | 0 | 0 |
| H | H | H | H | OCH₃ | 5-Methyl-furan-2-yl | H | 4 | 0 | 0 | 0 |
| H | H | H | H | OCH₃ | 2-Methyl-5-nitro-1-imidazolyl | H | 4 | 0 | 0 | 0 |
| H | H | H | H | OCH₃ | 5-Nitro-1-imidazolyl | H | 4 | 0 | 0 | 0 |

und die Salze dieser Verbindungen in den Tabellen.

Ein weiterer Gegenstand der Erfindung ist ein Verfahren zur Herstellung der Verbindungen der Formel I, worin R1, R2, R3, R4, R5, R6, R7, m, n, p und q die oben angegebenen Bedeutungen haben, und ihrer Salze.

Das Verfahren ist dadurch gekennzeichnet, daß man
a) Mercaptobenzimidazole der Formel II (siehe beigefügtes Formelblatt), worin R1, R2, R3 und R4 die oben angegebenen Bedeutungen haben, mit Picolinderivaten III (siehe beigefügtes Formelblatt), worin R5, R6, R7, m, p und q die oben angegebenen Bedeutungen haben und X eine geeignete Abgangsgruppe darstellt, umsetzt oder daß man
b) Verbindungen der Formel IV (siehe beigefügtes Formelblatt), worin R1, R2, R3, R4, R5, R7 und m die oben angegebenen Bedeutungen haben, n und p die Zahl 0 bedeuten und Y eine geeignete Abgangsgruppe darstellt, mit Thiolen R6-SH umsetzt und
(falls Verbindungen der Formel I mit n=1 bzw. p=1 und/oder q=1 die gewünschten Endprodukte sind), daß man anschließend die nach a) oder b) erhaltenen Verbindungen mit n=0 und/oder p=0 und/oder q=0 oxydiert, und/oder daß man erhaltene Verbindungen gewünschtenfalls anschließend in die Salze überführt und/oder daß man erhaltene Salze gewünschtenfalls anschließend in die freien Verbindungen überführt.

Bei der vorstehend aufgeführten Umsetzung können die Ausgangsverbindungen als solche oder gegebenenfalls in Form ihrer Salze eingesetzt werden.

Als geeignete Abgangsgruppen X bzw. Y seien beispielsweise Halogenatome, insbesondere Chlor, oder durch Veresterung (z.B. mit p-Toluolsulfonsäure) aktivierte Hydroxylgruppen genannt.

Die Umsetzung von II mit III erfolgt in geeigneten, vorzugsweise polaren protischen oder aprotischen Lösungsmitteln (wie Methanol, Ethanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 0° und 150°C liegen, wobei in Gegenwart von Protonenakzeptoren Temperaturen zwischen 20° und 80°C und ohne Protonenakzeptoren zwischen 60° und 120°C - insbesondere die Siedetemperatur der verwendeten Lösungsmittel - bevorzugt sind. Die Reaktionszeiten liegen zwischen 0,5 und 30 Stunden.

Die Umsetzung der Verbindungen IV mit den Thiolen R6-SH erfolgt auf ähnliche Weise wie die Umsetzung der Verbindungen II mit den Verbindungen III.

Die Oxidation der Sulfide (Verbindungen der Formel I mit n=0) zu den Sulfoxiden (Verbindungen der Formel I mit n=1) erfolgt unter den Bedingungen, wie sie dem Fachmann für die Oxidation von Sulfiden zu Sulfoxiden geläufig sind [siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980]. Als Oxidationsmittel kommen alle für die Oxidation von Sulfiden zu Sulfoxiden üblicherweise verwendeten Reagenzien in Frage, insbesondere Peroxysäuren, wie z.B. Peroxyessigsäure, Trifluorperoxyessigsäure, 3,5-Dinitroperoxybenzoesäure, Peroxymaleinsäure, Magnesiummonoperoxiphthalat oder bevorzugt m-Chlorperoxybenzoesäure.

Die Reaktionstemperatur liegt (je nach Reaktivität des Oxidationsmittels und Verdünnungsgrad) zwischen -70°C und der Siedetemperatur des verwendeten Lösungsmittels, bevorzugt jedoch zwischen -30° und +20°C. Als vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen 0° und 50°C durchgeführt wird. Die Reaktion wird zweckmäßigerweise in inerten Lösungsmitteln, z.B. aromatischen oder chlorierten Kohlenwasserstoffen, wie Benzol, Toluol, Dichlormethan oder Chloroform, vorzugsweise in Estern oder Ethern, wie Essigsäureethylester, Essigsäureisopropylester oder Dioxan, oder in Alkoholen, vorzugsweise Isopropanol, durchgeführt.

Die erfindungsgemäßen Sulfoxide sind optisch aktive Verbindungen. Je nach Art der Substituenten können noch weitere Chiralitätszentren im Molekül sein. Die Erfindung umfaßt daher sowohl die Enantiomeren und Diastereomeren als auch ihre Mischungen und Racemate. Die Enantiomeren können in an sich bekannter Weise (beispielsweise durch Herstellung und Trennung entsprechender diastereoisomerer Verbindungen) separiert werden (siehe z.B. WO92/08716).

Die Verbindungen II sind z.B. aus WO86/02646, EP 134 400 oder EP 127 763 bekannt. Die Verbindungen III mit p=0 bzw. q=0 können beispielsweise so wie in den nachfolgenden Beispielen beschrieben hergestellt werden.

Für Verbindungen III mit p=1 bzw. q=1 werden die entsprechenden 2-Hydroxymethyl-4-mercaptosubstituierten Pyridine beispielsweise mit m-Chlorperoxibenzoesäure zu den Sulfoxiden oxidiert und anschließend beispielsweise mit Thionylchlorid chloriert. Umsetzung mit 2-Mercaptobenzimidazolen liefert die Verbindungen der Formel I mit p=1 und q=1.

Je nach Art des Substituenten R6 werden die Sulfoxide q=1 auch bei der Oxidation zu den Sulfoxiden n=1 erhalten. Im übrigen können die jeweiligen Sulfide bzw. Sulfoxide durch Wahl geeigneter Ausgangsverbindungen bzw. durch Verwendung selektiver Oxidationsmittel hergestellt werden.

Die zur Herstellung von III benötigten Thiole R6-CₘH₂ₘ-SH können z.B. aus den entsprechenden Halogenverbindungen analog J. Med. Chem. 14 (1971) 349 hergestellt werden.

Die folgenden Beispiele erläutern die Erfindung näher, ohne sie einzuschränken. Die erfindungsgemäßen Verbindungen und die Ausgangsverbindungen können auf analoge Weise wie in den Beispielen beschrieben hergestellt werden.

### Beispiele

### Endprodukte

### 1. 2-{[[3-Methyl-4-(3-(5-methyl-1,3,4-thiadiazol-2-yl-thio)-propylthio)-2-pyridinyl]methyl]thio}-1H-benzimidazol

Zu einer Lösung von 5-Methyl-1,3,4-thiadiazol-2-thiol (0,16 g, 1,2 mMol) in 10 ml Ethanol und 1,4 ml IN Natronlauge wird 2-([[4-(3-Chlorpropylthio)-3-methyl-2-pyridinyl]methyl]thio}-1H-benzimidazol (0,36 g, 1,0 mMol) zugegeben und 20-30 h unter Rückfluß zum Sieden erhitzt. Man tropft 12 ml H₂O zu, läßt abkühlen, filtriert vom ausgefallenen Feststoff, wäscht mit Wasser und trocknet im Vakuum bei 50°C. Man erhält 0,38 g (83 % d.Th.) der Titelverbindung als farblosen Feststoff vom Schmp. 76-77°C.

### 2. 2-{[[3-Methyl-4-(3-(4-methyl-1,2,4-triazol-3-yl-thio)-propylthio)-2-pyridinyl]methyl]thio}-1H-benzimidazol

Nach der in Beispiel 1 angegebenen Arbeitsweise erhält man durch Umsetzung von 4-Methyl-1,2,4-triazol-3-thiol (0,19 g, 1,6 mMol) und 2-([[4-(3-Chlorpropylthio)-3-methyl-2-pyridinyl]methyl]thio}-1H-benzimidazol (0,36 g, 1,0 mMol) und 1,7 mMol IN Natronlauge die rohe Titelverbindung (wasserhaltig). Nach Kristallisation aus EE/Diisopropylether erhält man die Titelverbindung als farblosen Feststoff vom Schmp. 98-99°C (Ausb. 58 % d.Th.).

### 3. 2-{[[3-Methyl-4-(3-(1-methylimidazol-2-yl-thio)-propylthio)-2-pyridinyl]methyl]thio}-1H-benzimidazol

Nach der in Beispiel 1 angegebenen Arbeitsweise erhält man durch Umsetzung von 2-Mercapto-1-methylimidazol mit 2-([[4-(3-Chlorpropylthio)-3-methyl-2-pyridinyl]methyl]thio}-1H-benzimidazol und NaOH nach Kristallisation aus Essigester/Diisopropylether die Titelverbindung (68 % d.Th.) als farblosen Feststoff vom Schmp. 97-99°C.

### 4. 2-{[[4-[3-(3-Amino-1,2,4-triazol-5-yl-thio)-propylthiol-3-methyl-2-pyridinyl]methyl]thio}-1H-benzimidazol

Nach der in Beispiel 1 angegebenen Arbeitsweise erhält man durch Umsetzung von 3-Amino-5-mercapto-1,2,4-triazol mit 2-{[[4-(3-Chlorpropylthio)-3-methyl-2-pyridinyl]methyl]thio}-1H-benzimidazol und NaOH die Titelverbindung (82 % d.Th.) als farblosen Feststoff vom Schmp. 94-96°C.

### 5. 2-{[[4-[3-(5-Amino-1,3,4-thiadiazol-2-yl-thio)-propylthiol-3-methyl-2-pyridinyl]methyl]thio}-1H-benzimidazol

Nach der in Beispiel 1 angegebenen Arbeitsweise erhält man durch Umsetzung von 2-Amino-5-mercapto-1,3,4-thiadiazol mit 2-([[4-(3-Chlorpropylthio)-3-methyl-2-pyridinyl]methyl]thio}-1H-benzimidazol und NaOH die rohe Titelverbindung als Öl. Durch Auflösen in Isopropanol, Versetzen mit konz. Salzsäure (ca. 5 Equivalente), vollständiges Einengen und Verreiben mit Aceton erhält man die Titelverbindung als Trihydrochlorid; farbloser Feststoff; 105°C Gasentwicklung; über 240°C Zersetzung.

### 6. 2-{[[4-[3-Imidazol-2-yl-thio-propylthio]-3-methyl-2-pyridinyl]-methyl]thio}-1H-benzimidazol

Nach der in Beispiel 1 angegebenen Arbeitsweise erhält man durch Umsetzung von 2-Mercaptoimidazol mit 2-{[[4-(3-Chlorpropylthio)-3-methyl-2-pyridinyl]methyl]thio}-1H-benzimidazol und NaOH nach Umkristallisation aus Methanol/Toluol die Titelverbindung (62 % d.Th.); farbloser Feststoff vom Schmp. 195°C (Zers.).

### 7. 2-{[[3-Methyl-4-(3-(1,2,4-triazol-3-yl-thio)-propylthio)-2-pyridinyl]methyl]thio}-1H-benzimidazol

Nach der in Beispiel 1 angegebenen Arbeitsweise erhält man durch Umsetzung von 3-Mercapto-1,2,4-triazol mit 2-{[[4-(3-Chlorpropylthio)-3-methyl-2-pyridinyl]methyl]thio)-lH-benzimidazol und NaOH die Titelverbindung (76 % d.Th.); farbloser Feststoff vom Schmp. 80-82°C.

### 8. 2-{[[3-Methyl-4-(3-(1-methyl-tetrazol-5-yl-thio)-propylthio)-2-pyridinyl]methyl]thio}-1H-benzimidazol

Nach der in Beispiel 1 angegebenen Arbeitsweise erhält man durch Umsetzung von 1-Methyl-5-mercaptotetrazol mit 2-{[[4-(3-Chlorpropylthio)-3-methyl-2-pyridinyl]methyl]thio)}-1H-benzimidazol und NaOH die Titelverbindung (68 % d.Th.); farbloser Feststoff vom Schmp. 99-103°C.

### 9. 2-{[[3-Methyl-4-(3-(thiazol-2-yl-thio)-propylthio)-2-pyridinyl]methyl]thio}-1H-benzimidazol

Nach der in Beispiel 1 angegebenen Arbeitsweise erhält man durch Umsetzung von 2-Mercaptothiazol mit 2-([[4-(3-Chlorpropylthio)-3-methyl-2-pyridinyl]methyl]thio}-1H-benzimidazol und NaOH die Titelverbindung (63 % d.Th.); farbloser Feststoff vom Schmp. 121-123°C.

### 10. 2-{[[3-Methyl-4-(3-(pyridin-2-yl-thio)-propylthio)-2-pyridinyl]methyl]thio}-1H-benzimidazol

Nach der in Beispiel 1 angegebenen Arbeitsweise erhält man durch Umsetzung von 2-Mercaptopyridin mit 2-{[[4-(3-Chlorpropylthio)-3-methyl-2-pyridinyl]methyl]thio}-1H-benzimidazol und NaOH die Titelverbindung (87 % d.Th.); farbloser Feststoff vom Schmp. 97-99°C.

### 11. 2-{[[3-Methyl-4-(3-(pyrimidin-2-yl-thio)-propylthio)-2-pyridinyl]methyl]thio}-1H-benzimidazol

Nach der in Beispiel 1 angegebenen Arbeitsweise erhält man durch Umsetzung von 2-Mercaptopyrimidin mit 2-{[[4-(3-Chlorpropylthio)-3-methyl-2-pyridinyl]methyl]thio}-1H-benzimidazol und NaOH die Titelverbindung als Monohydrat (92 % d.Th.); farbloser Feststoff vom Schmp. 118-119°C.

### 12. 2-{[[4-[(3-Benzimidazol-2-yl-thio)-propylthio]-3-methyl-2-pyridinyl]methyl]thio]-1H-benzimidazol

Nach der in Beispiel 1 angegebenen Arbeitsweise erhält man durch Umsetzung von 2-Mercaptobenzimidazol mit 2-{[[4-(3-Chlorpropylthio)-3-methyl-2-pyridinyl]methyl]thio}-1H-benzimidazol und NaOH die Titelverbindung (62 % d.Th.); farbloser Feststoff vom Schmp. 177-180°C (Zers.).

### 13. 2-{[[3-Methyl-4-(3-phenylthio-propylthio)-2-pyridinyl]methyl]thio}-1H-benzimidazol

Nach der in Beispiel 1 angegebenen Arbeitsweise erhält man durch Umsetzung von Thiophenol mit 2-([[4-(3-Chlorpropylthio)-3-methyl-2-pyridinyl]methyl]thio}-1H-benzimidazol und NaOH die Titelverbindung (95 % d.Th.); farbloser Feststoff vom Schmp. 141-143°C.

### 14. 2-{[[3-Methyl-4-(3-(pyridin-1-oxo-2-yl-thio)-propylthio)-2-pyridinyl]methyl]thio}-1H-benzimidazol-dihydrochlorid

Nach der in Beispiel 1 angegebenen Arbeitsweise erhält man durch Umsetzung von 2-Mercaptopyridin-N-oxid mit 2-([[4-(3-Chlorpropylthio)-3-methyl-2-pyridinyl]methyl]thio}-1H-benzimidazol und NaOH die Titelverbindung als zähes Öl. Man extrahiert mit Dichlormethan, trocknet über Kaliumcarbonat, engt vollständig ein, löst den Rückstand in Isopropanol, versetzt mit 3 Equivalenten konz. Salzsäure und destilliert an. Der ausgefallene Feststoff wird filtriert und über KOH im Vakuum getrocknet. Man erhält die Titelverbindung (66 % d.Th.); farbloser Feststoff vom Schmp. 71-73°C (Zers.).

### 15. 2-{[[3-Methyl-4-(2-(pyridin-2-yl-thio)-ethylthio)-2-pyridinyl]methyl]thio}-1H-benzimidazol

Nach der in Beispiel 1 angegebenen Arbeitsweise erhält man durch Umsetzung von 2-Mercaptopyridin mit 2-([[4-(2-Chlorethylthio)-3-methyl-2-pyridinyl]methyl]thio}-1H-benzimidazol und Natronlauge nach Kristallisation aus Isopropanol die Titelverbindung (72 % d.Th.) als farblosen Feststoff vom Schmp. 145-147°C.

### 16. 2-{[[3-Methyl-4-[3-(4-methyl-pyrimidin-2-yl-thio)-propylthio]-2-pyridinyl]methyl]thio}-1H-benzimidazol

Nach der in Beispiel 1 beschriebenen Arbeitsweise erhält man durch Umsetzung mit 2-Mercapto-4-methylpyrimidin die Titelverbindung; beiger Feststoff; Schmp. 70-72°C; Ausb. 77 % d.Th.

### 17. 2-{[[4-[3-(1-(2-Dimethylaminoethyl)-tetrazol-5-yl-thio)-propylthiol-3-methyl-2-pyridinyl]methyl]thio}-1H-benzimidazol-trihydrochlorid

Nach der in den Beispielen 14 und 1 beschriebenen Arbeitsweise erhält man durch Umsetzung mit 1-(2-Dimethylamino)-2-mercaptotetrazol die Titelverbindung; Schmp. 131-133°C Zers.; Ausb. 40 % d.Th.

### 18. 2-{[[3-Methyl-4-(3-(1,3,4-thiadiazol-2-yl-thio)-propylthio)-2-pyridinyl]methyl]thio}-1H-benzimidazol-trihydrochlorid

Nach der in den Beispielen 14 und 1 beschriebenen Arbeitsweise erhält man durch Umsetzung mit 2-Mercapto-1,3,4-thiadiazol die Titelverbindung; Schmp. 167-170°C Zers.; Ausb. 53 % d.Th.

### 19. 2-{[[3-Methyl-4-(3-(pyridin-4-yl-thio)-propylthio)-2-pyridinyl]methyl]thio)-1H-benzimidazol

Nach der in Beispiel 1 beschriebenen Arbeitsweise erhält man durch Umsetzung mit 4-Mercaptopyridin die Titelverbindung; farbloses Pulver; Schmp.: 164-166°C; Ausb. 92 % d.Th.

### 20. 2-{[[3-Methoxy-4-(3-(1-methyl-tetrazol-5-yl-thio)-propylthio)-2-pyridinyl]methyl]thio}-1H-benzimidazol-dihydrochlorid

Nach der in den Beispielen 14 und 1 beschriebenen Arbeitsweise erhält man durch Umsetzung von 2-([[4-(3-Chlorpropylthio)-3-methoxy-2-pyridinyl]methyl]thio)-lH-benzimidazol mit 1-Methyl-5-mercaptotetrazol die Titelverbindung als farbloses Kristallisat; Schmp. 135°C Zers.; Ausb. 84 % d.Th.

### 21. 2-{[[3-Methoxy-4-(3-(pyrimidin-2-yl-thio)-propylthio)-2-pyridinyl]methyl]thio}-1H-benzimidazol

Nach der in Beispiel 1 beschriebenen Arbeitsweise erhält man durch Umsetzung von 2-Mercaptopyrimidin mit 2-{[[4-(3-Chlorpropylthio)-3-methoxy-2-pyridinyl]methyl]thio}-1H-benzimidazol die Titelverbindung als beiges Pulver; Schmp. 88-90°C; Ausb. 77 % d.Th.

### 22. Natrium-5-{5-[2-(1H-benzimidazol-2-yl-thiomethyl)-3-methyl-4-pyridinyl]-1,5-dithiapent-1-yl}-tetrazol-1-acetat

Nach der in Beispiel 1 beschriebenen Arbeitsweise erhält man durch Umsetzung mit 2-Mercaptotetrazol-1-essigsäure und Natronlauge, nach Fällung mit Aceton und Umkristallisation aus Methanol/Diisopropylether die Titelverbindung; beiges Pulver; Schmp. ab 180°C Zers.; Ausb. 34 % d.Th.

### 23. 2-{[[3-Methyl-4-(2-(1-methyl-tetrazol-5-yl-thio)-ethylthio)-2-pyridinyl]methyl]thio}-1H-benzimidazol

Nach der in Beispiel 1 angegebenen Arbeitsweise erhält man durch Umsetzung von 1-Methyl-5-mercaptotetrazol mit 2-{[[4-(2-Chlorethylthio)-3-methyl--2-pyridinyl]methyl]thio)}-1H-benzimidazol und NaOH die Titelverbindung (82 % d.Th.) als farblosen Feststoff. Schmp. 204-208°C.

### 24. 2-{[[3-Methyl-4-(4-(1-methyl-tetrazol-5-yl-thio)-butylthio)-2-pyridinyl]methyl]thio}-1H-benzimidazol

Nach der in Beispiel 1 angegebenen Arbeitsweise erhält man durch Umsetzung von 1-Methyl-5-mercaptotetrazol mit 2-{[[4-(4-Chlorbutylthio)-3-methyl--2-pyridinyl]methyl]thio)}-1H-benzimidazol und NaOH die Titelverbindung (48 % d.Th.) als farblosen Feststoff. Schmp. 208-210°C.

### Ausgangsverbindungen

### A1. 2-{[[4-(3-Chlorpropylthio)-3-methyl-2-pyridinyl]methyl]thio}-1H-benzimidazol

Zu einer Lösung von 2-Mercapto-lH-benzimidazol (1,5 g/10 mMol) in 40 ml Ethanol und 21 ml 1 N Natronlauge wird ein Äquivalent 2-Chlormethyl-4-(3-chlorpropylthio)-3-methylpyridin-hydrochlorid (gelöst in 10 ml Wasser) innerhalb von 20 Min. bei 40°C zugetropft. Man rührt anschließend 2 - 3 h bei 50-60°C und weitere 3 - 4 h bei Raumtemperatur, destilliert Ethanol am Rotationsverdampfer (1 kPa/40°C) ab, extrahiert 3 mal mit je 20 ml Dichlormethan, wäscht mit 0,1 N Natronlauge, trocknet über Kaliumcarbonat und engt im Vakuum vollständig ein. Zur Reinigung wird das Rohprodukt an Kieselgel chromatographiert (Dichlormethan 20:1 bis 3:1); die gesammelten reinen Fraktionen werden gemeinsam im Vakuum eingeengt und aus Dichlormethan/Diisopropylether zur Kristallisation gebracht. Anschließend wird aus Methanol/Toluol umkristallisiert. Ausbeute: 2,67 g (74 %) der Titelverbindung als farbloser Feststoff vom Schmp. 112-114°C.

### A2. 2-Chlormethyl-4-(3-chlorpropylthio)-3-methylpyridin-hydrochlorid

### a) 2,3-Dimethyl-4-(3-hydroxypropylthio)pyridin-N-oxid

Zu 50 ml trockenem N-Methylpyrrolidon (NMP) werden 6 g (60 %iges) NaH portionsweise zugegeben, es wird 15 Min. gerührt, 9,5 g (0,11 Mol) 3-Hydroxypropylmercaptan werden innerhalb von 20 Min. zudosiert und es wird erneut 30 Min. bis zur Beendigung der Gasentwicklung gerührt. Anschließend tropft man innerhalb von 20 Min. eine Lösung von 14,4 g (0,1 Mol) 4-Chlor-2,3-dimethylpyridin-N-oxid in 100 ml NMP zu, rührt die Reaktionsmischung 1 h bei Raumtemperatur, anschließend 1 h bei 70°C und danach noch 1 h bei 100°C.

Nach beendeter Umsetzung läßt man abkühlen, verdünnt mit 500 ml Wasser und extrahiert 4 mal mit je 300 ml Dichlormethan. Die vereinigten organischen Phasen werden mit Wasser gewaschen, über Magnesiumsulfat getrocknet und eingeengt. Der ölige Rückstand [10,0 g rohes 2,3-Dimethyl-4-(3-hydroxypropylthio)-pyridin-N-oxid] wird direkt in der Folgestufe eingesetzt.

### b) 2-Hydroxymethyl-4-(3-hydroxypropylthio)-3-methylpyridin

Man löst das unter a) erhaltene gelbe Öl in 100 ml Essigsäureanhydrid, und rührt 2 h bei 100°C. Nach Einengen im Vakuum wird der braune, ölige Rückstand in einer Kugelrohrdestillationsapparatur destilliert und ohne Reinigung weiter umgesetzt.

Das ölige Destillat wird in 100 ml 2 N Natronlauge und 100 ml Isopropanol 2 h unter Rühren auf Rückflußtemperatur erhitzt, Isopropanol abdestilliert, der Rückstand 3 mal mit je 100 ml Dichlormethan extrahiert, die vereinigten organischen Phasen mit Wasser gewaschen, über Kaliumcarbonat getrocknet und im Vakuum eingeengt. Man erhält 5,0 g 2-Hydroxymethyl-4-(3-hydroxypropylthio)-3-methylpyridin, das ohne Reinigung weiter umgesetzt wird.

### c) 2-Chlormethyl-4-(3-chlorpropylthio)-3-methylpyridin-hydrochlorid

5,0 g des Öls aus Beispiel b) werden in Dichlormethan (100 ml) gelöst, 4 Äquivalente Thionylchlorid zugetropft und 20 h bei Raumtemperatur gerührt. Man engt vollständig ein und erhält 4,5 g der Titelverbindung als öligen, allmählich kristallisierenden Rückstand, der gewünschtenfalls auch als Lösung in Ethanol direkt zur Umsetzung mit gegebenenfalls substituierten 2-Mercaptobenzimidazolen verwendet werden kann.

### B1. 2-{[[4-(2-Chlorethylthio)-3-methyl-2-pyridinyl]methyl]thio}-1H-benzimidazol

Nach der in Beispiel A1. angegebenen Arbeitsweise erhält man durch Umsetzung von 2-Mercapto-1H-benzimidazol mit 4-(2-Chlorethylthio)-2-chlormethyl-3-methylpyridin-hydrochlorid und NaOH nach Kristallisation aus Essigester die Titelverbindung (62 % d.Th.) als farblosen Feststoff vom Schmp. 178-180°C.

### B2. 4-(2-Chlorethylthio)-2-chlormethyl-3-methylpyridin-hydrochlorid

### a) 2,3-Dimethyl-4-(2-hydroxyethylthio)pyridin-N-oxid

Nach der in Beispiel A2.a) angegebenen Arbeitsweise erhält man durch Umsetzung von 4-Chlor-2,3-dimethylpyridin-N-oxid mit 2-Mercaptoethanol und Natriumhydrid die Titelverbindung als öligen Rückstand, der ohne weitere Reinigung in der Folgestufe eingesetzt wird.

### b) 4-(2-Hydroxyethylthio)-2-hydroxymethyl-3-methylpyridin

Nach der in Beispiel A2.b) angegebenen Arbeitsweise erhält man durch Umsetzung des unter a) erhaltenen Öls mit Essigsäureanhydrid und anschließender Verseifung mit NaOH die Titelverbindung als öligen Rückstand, der ohne weitere Reinigung in der Folgestufe eingesetzt wird.

### c) 4-(2-Chlorethylthio)-2-chlormethyl-3-methylpyridin-hydrochlorid

Nach der in Beispiel A2.c) angegebenen Arbeitsweise erhält man durch Umsetzung des unter b) erhaltenen Öls mit Thionylchlorid die Titelverbindung als öligen Rückstand, der als Lösung in Ethanol direkt zur Umsetzung mit 2-Mercaptobenzimidazol eingesetzt wird.

### C1. 2-{[[4-(4-Chlorbutylthio)-3-methyl-2-pyridinyl]methyl]thio)}-1H-benzimidazol

Nach der in Beispiel A1. angegebenen Arbeitsweise erhält man durch Umsetzung von 2-Mercapto-1H-benzimidazol mit 4-(4-Chlorbutylthio)-2-chlormethyl-3-methylpyridin-hydrochlorid und NaOH nach Kristallisation aus Essigester/Diisopropylether die Titelverbindung (82 % d.Th.) als schwachgelben Feststoff vom Schmp. 151-153°C.

### C2. 4-(4-Chlorbutylthio)-2-chlormethyl-3-methylpyridin-hydrochlorid

### a) 2,3-Dimethyl-4-(4-hydroxybutylthio)pyridin-N-oxid

Nach der in Beispiel A2.a) angegebenen Arbeitsweise erhält man durch Umsetzung von 4-Chlor-2,3-dimethylpyridin-N-oxid mit 4-Mercaptobutanol und Natriumhydrid die Titelverbindung als öligen Rückstand, der ohne weitere Reinigung in der Folgestufe eingesetzt wird.

### b) 4-(4-Hydroxybutylthio)-2-hydroxymethyl-3-methylpyridin

Nach der in Beispiel A2.b) angegebenen Arbeitsweise erhält man durch Umsetzung des unter a) erhaltenen Öls mit Essigsäureanhydrid und anschließender Verseifung mit NaOH die Titelverbindung als öligen Rückstand, der ohne weitere Reinigung in der Folgestufe eingesetzt wird.

### c) 4-(4-Chlorbutylthio)-2-chlormethyl-3-methylpyridin-hydrochlorid

Nach der in Beispiel A2.c) angegebenen Arbeitsweise erhält man durch Umsetzung des unter b) erhaltenen Öls mit Thionylchlorid die Titelverbindung als öligen Rückstand, der als Lösung in Ethanol direkt zur Umsetzung mit 2-Mercaptobenzimidazol eingesetzt wird.

### D1. 2-{[[4-(3-Chlorpropylthio)-3-methoxy-2-pyridinyl]methyl]thio}-1H-benzimidazol-dihydrochlorid

2-Mercapto-1H-benzimidazol (10 g) und 2-Chlormethyl-4-(3-chlorpropylthio)-3-methoxypyridin-hydrochlorid (1 Equivalent) werden in 150 ml Isopropanol und 15 ml Wasser 5 h bei 80°C gerührt, abgekühlt, vom ausgefallenen Feststoff filtriert und aus Isopropanol/Wasser umkristallisiert. Man erhält die Titelverbindung als hellbraunes Pulver; Schmp. 117-119°C (Zers.); Ausb.: 67 % d.Th.

### D2. 2-Chlormethyl-4-(3-chlorpropylthio)-3-methoxy-pyridin-hydrochlorid

Nach der im Beispiel A2 a, b, c beschriebenen Arbeitsweise erhält man ausgehend von 4-Chlor-3-methoxy-2-methylpyridin-N-oxid die Titelverbindung als langsam kristallisierendes Öl, das direkt weiter umgesetzt wird.

### Gewerbliche Anwendbarkeit

Die ausgezeichnete Wirksamkeit von Verbindungen der Formel I und ihren Salzen gegen Helicobacter-Bakterien gestattet ihren Einsatz in der Humanmedizin als Wirkstoffe für die Behandlung von Krankheiten, die auf Helicobacter-Bakterien beruhen.

Ein weiterer Gegenstand der Erfindung ist daher ein Verfahren zur Behandlung von Säugern, insbesondere Menschen, die an Krankheiten erkrankt sind, die auf Helicobacter-Bakterien beruhen. Das Verfahren ist dadurch gekennzeichnet, daß man dem erkrankten Individuum eine therapeutisch wirksame und pharmakologisch verträgliche Menge einer oder mehrerer Verbindungen der Formel I und/oder ihrer pharmakologisch verträglichen Salze verabreicht.

Gegenstand der Erfindung sind außerdem die Verbindungen der Formel I und ihre pharmakologisch verträglichen Salze zur Anwendung bei der Behandlung von Krankheiten, die auf Helicobacter-Bakterien beruhen.

Ebenso umfaßt die Erfindung die Verwendung von Verbindungen der Formel I und ihren pharmakologisch verträglichen Salzen bei der Herstellung von Arzneimitteln, die zur Bekämpfung solcher Krankheiten eingesetzt werden, die auf Helicobacter-Bakterien beruhen.

Ein weiterer Gegenstand der Erfindung sind Arzneimittel zur Bekämpfung von Helicobacter-Bakterien, die eine oder mehrere Verbindungen der allgemeinen Formel I und/oder ihre pharmakologisch verträglichen Salze enthalten.

Von den Helicobacter-Stämmen, gegenüber denen sich die Verbindungen der Formel I als wirksam erweisen, sei insbesondere der Stamm Helicobacter pylori erwähnt.

Die Arzneimittel werden nach an sich bekannten, dem Fachmann geläufigen Verfahren hergestellt. Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen z.B. in Form von Tabletten, Dragees, Kapseln, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder Permeationspromotoren und Komplexbildner (z.B. Cyclodextrine) verwendet werden.

Die Wirkstoffe können beispielsweise parenteral (z.B. intravenös) oder insbesondere oral appliziert werden.

Im allgemeinen werden in der Humanmedizin die Wirkstoffe in einer Tagesdosis von etwa 0,2 bis 50, vorzugsweise 1 bis 30 mg/kg Körpergewicht, gegebenenfalls in Form mehrerer, vorzugsweise 2 bis 6 Einzelgaben zur Erzielung des gewünschten Ergebnisses verabreicht.

In diesem Zusammenhang ist als erfindungswesentlicher Aspekt besonders zu erwähnen, daß sich die Verbindungen der Formel I, in denen n die Zahl 0 bedeutet, gegenüber Helicobacter-Bakterien bereits bei Verabfolgung solcher Dosen als wirksam erweisen, die unterhalb der Dosen liegen, die zur Erzielung einer - therapeutischen Zwecken genügenden - Magensäuresekretionshemmung eingesetzt werden müßten.

Verbindungen der Formel I, in denen n die Zahl 1 bedeutet, besitzen - neben ihrer Wirksamkeit gegen Helicobacter-Bakterien - auch eine ausgeprägte magensäuresekretionshemmende Wirkung. Entsprechend können diese Verbindungen auch zur Behandlung solcher Krankheiten eingesetzt werden, die auf einer erhöhten Magensäuresekretion beruhen.

Die erfindungsgemäßen Verbindungen können auch in fixer oder freier Kombination zusammen mit einer die Magensäure neutralisierenden und/oder die Magensäuresekretion hemmenden Substanz und/oder mit einer für die klassische Bekämpfung des Helicobacter pylori geeigneten Substanz verabfolgt werden.

Als die Magensäure neutralisierende Substanzen seien beispielsweise Natriumhydrogencarbonat oder andere Antacida (wie Aluminiumhydroxid, Magnesiumaluminat oder Magaldrat) genannt. Als die Magensäuresekretion hemmende Substanzen seien beispielsweise H₂-Blocker (z.B. Cimetidin, Ranitidin), H+/K+-ATPase-Hemmstoffe (z.B. Lansoprazol, Omeprazol oder insbesondere Pantoprazol) sowie sogenannte periphere Anticholinergika (z.B. Pirenzepin, Telenzepin) genannt.

Als für die klassische Bekämpfung des Helicobacter pylori geeignete Substanzen seien insbesondere antimikrobiell wirksame Substanzen wie beispielsweise Penicillin G, Gentamycin, Erythromycin, Nitrofurazon, Tinidazol, Nitrofurantoin, Furazolidon, Metronidazol und insbesondere Amoxycillin, oder aber auch Wismutsalze wie z.B. Wismutcitrat genannt.

### Biologische Untersuchungen

Die Verbindungen der Formel I wurden bezüglich ihrer Wirksamkeit gegenüber Helicobacter pylori in Anlehnung an die von Tomoyuki Iwahi et al. (Antimicrobial Agents and Chemotherapy, 1991, 490-496) beschriebene Methodik unter Verwendung von Columbia-agar (Oxoid) und bei einer Wachstumsperiode von 4 Tagen untersucht. Für die untersuchten Verbindungen ergaben sich hierbei die in der nachfolgenden Tabelle 2 aufgeführten ca. MIC 50-Werte (die angegebenen Nummern der Verbindungen stimmen mit den Verbindungsnummern in der Beschreibung überein).

**TABELLE 2**

| Verbindung Nr. | ca. MIC 50-Wert (µg/ml) |
|---|---|
| 1 | 0,1 |
| 2 | 0,05 |
| 3 | 0,05 |
| 5 | 0,5 |
| 6 | 0,1 |
| 8 | 0,05 |
| 9 | 0,05 |
| 10 | 0,05 |
| 11 | 0,05 |
| 12 | 0,05 |
| 13 | 0,05 |
| 16 | 0,05 |
| 18 | 0,05 |
| 19 | 0,05 |
| 24 | 0,05 |

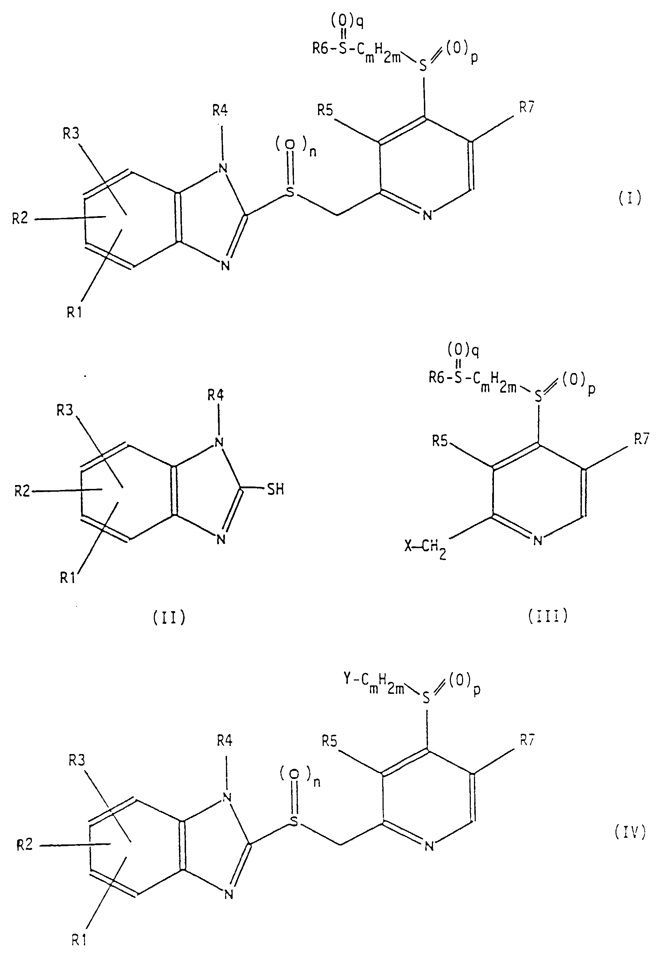

## Patentansprüche

1. Verbindungen der Formel I, 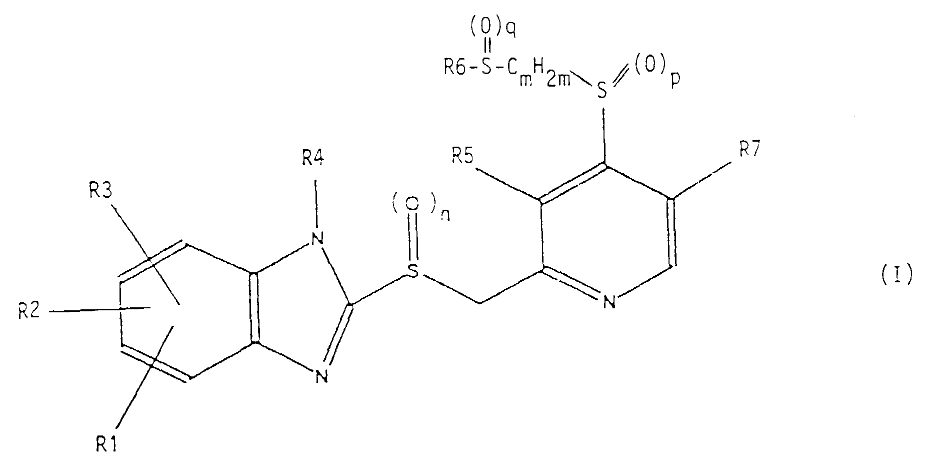 worin
R1 Wasserstoff, 1-4C-Alkyl oder 1-4C-Alkoxy bedeutet,
R2 Wasserstoff, 1-4C-Alkyl, 1-4C-Alkoxy, Halogen, Trifluormethyl, ganz oder überwiegend durch Fluor substituiertes 1-4C-Alkoxy, Chlordifluormethoxy, 2-Chlor-1,1,2-trifluorethoxy oder gemeinsam mit R3 gewünschtenfalls ganz oder teilweise durch Fluor substituiertes 1-2C-Alkylendioxy oder Chlortrifluorethylendioxy bedeutet,
R3 Wasserstoff, ganz oder überwiegend durch Fluor substituiertes 1-4C-Alkoxy, Chlordifluormethoxy, 2-Chlor-1,1,2-trifluorethoxy oder gemeinsam mit R2 gewünschtenfalls ganz oder teilweise durch Fluor substituiertes 1-2C-Alkylendioxy oder Chlortrifluorethylendioxy bedeutet,
R4 Wasserstoff oder 1-4C-Alkyl bedeutet,
R5 Wasserstoff, 1-4C-Alkyl oder 1-4C-Alkoxy bedeutet,
R6 einen durch R8 und R9 substituierten Cyclus oder Bicyclus bedeutet, der ausgewählt ist aus der Gruppe bestehend aus Benzol, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin und Benzimidazol,
R7 Wasserstoff, 1-4C-Alkyl oder 1-4C-Alkoxy bedeutet,
R8 Wasserstoff, 1-4C-Alkyl, Hydroxy, 1-4C-Alkoxy, Halogen, Nitro, Carboxy, 1-4C-Alkoxycarbonyl, Guanidino, durch R10 substituiertes 1-4C-Alkyl oder -N(R11)R12 bedeutet,
R9 Wasserstoff, 1-4C-Alkyl, 1-4C-Alkoxy, Fluor oder Trifluormethyl bedeutet,
R10 Hydroxy, 1-4C-Alkoxy, Carboxy, 1-4C-Alkoxycarbonyl oder -N(R11)R12 bedeutet, wobei
R11 Wasserstoff, 1-4C-Alkyl oder -CO-R13 und
R12 Wasserstoff oder 1-4C-Alkyl bedeutet, oder wobei
R11 und R12 zusammen und unter Einschluß des Stickstoffatoms, an das beide gebunden sind, einen Piperidino- oder Morpholinorest darstellen,
R13 Wasserstoff, 1-4C-Alkyl oder 1-4C-Alkoxy bedeutet,
m eine Zahl von 2 bis 7 bedeutet,
n die Zahl 0 oder 1 bedeutet,
p die Zahl 0 oder 1 bedeutet und
q die Zahl 0 oder 1 bedeutet,
und ihre Salze.

2. Verbindungen der Formel I nach Anspruch 1, worin
R1 Wasserstoff, 1-4C-Alkyl oder 1-4C-Alkoxy bedeutet,
R2 Wasserstoff, 1-4C-Alkyl, 1-4C-Alkoxy, Halogen, Trifluormethyl, ganz oder überwiegend durch Fluor substituiertes 1-4C-Alkoxy, Chlordifluormethoxy, 2-Chlor-1,1,2-trifluorethoxy oder gemeinsam mit R3 gewünschtenfalls ganz oder teilweise durch Fluor substituiertes 1-2C-Alkylendioxy oder Chlortrifluorethylendioxy bedeutet,
R3 Wasserstoff, ganz oder überwiegend durch Fluor substituiertes 1-4C-Alkoxy, Chlordifluormethoxy, 2-Chlor-1,1,2-trifluorethoxy oder gemeinsam mit R2 gewünschtenfalls ganz oder teilweise durch Fluor substituiertes 1-2C-Alkylendioxy oder Chlortrifluorethylendioxy bedeutet,
R4 Wasserstoff oder 1-4C-Alkyl bedeutet,
R5 Wasserstoff, 1-4C-Alkyl oder 1-4C-Alkoxy bedeutet,
R6 einen durch R8 und R9 substituierten Cyclus oder Bicyclus bedeutet, der ausgewählt ist aus der Gruppe bestehend aus Benzol, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin und Benzimidazol,
R7 Wasserstoff, 1-4C-Alkyl oder 1-4C-Alkoxy bedeutet,
R8 Wasserstoff, 1-4C-Alkyl, Hydroxy, 1-4C-Alkoxy, Halogen, Guanidino, durch R10 substituiertes 1-4C-Alkyl oder -N(R11)R12 bedeutet,
R9 Wasserstoff, 1-4C-Alkyl, 1-4C-Alkoxy, Fluor oder Trifluormethyl bedeutet,
R10 Hydroxy, 1-4C-Alkoxy, Carboxy oder -N(R11)R12 bedeutet, wobei
R11 Wasserstoff, 1-4C-Alkyl oder -CO-R13 und
R12 Wasserstoff oder 1-4C-Alkyl bedeutet, oder wobei
R11 und R12 zusammen und unter Einschluß des Stickstoffatoms, an das beide gebunden sind, einen Piperidino- oder Morpholinorest darstellen,
R13 Wasserstoff, 1-4C-Alkyl oder 1-4C-Alkoxy bedeutet,
m eine Zahl von 2 bis 7 bedeutet,
n die Zahl 0 oder 1 bedeutet,
p die Zahl 0 oder 1 bedeutet und
q die Zahl 0 oder 1 bedeutet,
und ihre Salze.

3. Verbindungen der Formel I nach Anspruch 1, worin R8 Nitro, Carboxy oder 1-4C-Alkoxycarbonyl bedeutet, und/oder worin R10 1-4C-Alkoxycarbonyl bedeutet, und ihre Salze.

4. Verbindungen der Formel I nach Anspruch 1, in denen
R1 Wasserstoff bedeutet,
R2 Wasserstoff, Halogen, Methoxy, Difluormethoxy oder Trifluormethyl bedeutet,
R3 Wasserstoff bedeutet,
R4 Wasserstoff bedeutet,
R5 Wasserstoff, 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Isoxazol, Thiazol, Imidazol, Triazol, Tetrazol, Thiadiazol, Pyridin, Pyridin-N-oxid und Pyrimidin,
R7 Wasserstoff oder 1-4C-Alkyl bedeutet,
R8 Wasserstoff, 1-4C-Alkyl, durch R10 substituiertes 1-4C-Alkyl oder Amino bedeutet,
R9 Wasserstoff oder 1-4C-Alkyl bedeutet,
R10 Hydroxy, Carboxy oder -N(R11)R12 bedeutet, wobei
R11 Wasserstoff, 1-4C-Alkyl oder -CO-R13 und
R12 Wasserstoff oder 1-4C-Alkyl bedeutet, oder wobei
R11 und R12 zusammen und unter Einschluß des Stickstoffatoms, an das beide gebunden sind, einen Piperidinorest darstellen,
R13 Wasserstoff, 1-4C-Alkyl oder 1-4C-Alkoxy bedeutet,
m eine Zahl von 2 bis 4 bedeutet,
n die Zahl 0 oder 1 bedeutet,
p die Zahl 0 bedeutet und
q die Zahl 0 bedeutet,
und ihre Salze.

5. Verbindungen der Formel I nach Anspruch 1, in denen
R1 Wasserstoff bedeutet,
R2 Wasserstoff, Halogen oder Methoxy bedeutet,
R3 Wasserstoff bedeutet,
R4 Wasserstoff bedeutet,
R5 Wasserstoff, 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Isoxazol, Thiazol, Imidazol, Triazol, Tetrazol, Thiadiazol, Pyridin, Pyridin-N-oxid, Pyrimidin und Benzimidazol,
R7 Wasserstoff oder 1-4C-Alkyl bedeutet,
R8 Wasserstoff, 1-4C-Alkyl, Hydroxy, Nitro, Guanidino, Carboxy, 1-4C-Alkoxycarbonyl, durch R10 substituiertes 1-4C-Alkyl oder Amino bedeutet,
R9 Wasserstoff oder 1-4C-Alkyl bedeutet,
R10 Hydroxy, Carboxy, 1-4C-Alkoxycarbonyl oder -N(R11)R12 bedeutet, wobei
R11 Wasserstoff, 1-4C-Alkyl oder -CO-R13 und
R12 Wasserstoff oder 1-4C-Alkyl bedeutet, oder wobei
R11 und R12 zusammen und unter Einschluß des Stickstoffatoms, an das beide gebunden sind, einen Piperidinorest darstellen,
R13 Wasserstoff, 1-4C-Alkyl oder 1-4C-Alkoxy bedeutet,
m eine Zahl von 2 bis 4 bedeutet,
n die Zahl 0 oder 1 bedeutet,
p die Zahl 0 bedeutet und
q die Zahl 0 bedeutet,
und ihre Salze.

6. Verbindungen der Formel I nach Anspruch 1, in denen
R1 Wasserstoff bedeutet,
R2 Wasserstoff oder Methoxy bedeutet,
R3 Wasserstoff bedeutet,
R4 Wasserstoff bedeutet,
R5 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Thiazol, Triazol, Tetrazol, Thiadiazol und Pyridin,
R7 Wasserstoff bedeutet,
R8 Wasserstoff, 1-4C-Alkyl oder durch R10 substituiertes Methyl oder Ethyl bedeutet,
R9 Wasserstoff oder 1-4C-Alkyl bedeutet,
R10 Hydroxy, Carboxy oder -N(R11)R12 bedeutet, wobei
R11 1-4C-Alkyl und
R12 1-4C-Alkyl bedeutet, oder wobei
R11 und R12 zusammen und unter Einschluß des Stickstoffatoms, an das beide gebunden sind, einen Piperidinorest darstellen,
m die Zahl 2 oder 3 bedeutet,
n die Zahl 0 bedeutet,
p die Zahl 0 bedeutet und
q die Zahl 0 bedeutet,
und ihre Salze.

7. Verbindungen der Formel I nach Anspruch 1, in denen
R1 Wasserstoff bedeutet,
R2 Wasserstoff, Fluor oder Methoxy bedeutet,
R3 Wasserstoff bedeutet,
R4 Wasserstoff bedeutet,
R5 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin, Pyrimidin und Benzimidazol,
R7 Wasserstoff bedeutet,
R8 Wasserstoff, 1-4C-Alkyl, Hydroxy, Nitro, Guanidino, Carboxy, 1-4C-Alkoxycarbonyl oder durch R10 substituiertes Methyl oder Ethyl bedeutet,
R9 Wasserstoff oder 1-4C-Alkyl bedeutet,
R10 Hydroxy, Carboxy oder -N(R11)R12 bedeutet, wobei
R11 1-4C-Alkyl und
R12 1-4C-Alkyl bedeutet, oder wobei
R11 und R12 zusammen und unter Einschluß des Stickstoffatoms, an das beide gebunden sind, einen Piperidinorest darstellen,
m eine Zahl von 2 bis 4 bedeutet,
n die Zahl 0 bedeutet,
p die Zahl 0 bedeutet und
q die Zahl 0 bedeutet,
und ihre Salze.

8. Verbindungen der Formel I nach Anspruch 1, in denen
R1 Wasserstoff bedeutet,
R2 Wasserstoff bedeutet,
R3 Wasserstoff bedeutet,
R4 Wasserstoff bedeutet,
R5 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Thiazol, Triazol, Tetrazol, Thiadiazol und Pyridin,
R7 Wasserstoff bedeutet,
R8 Wasserstoff, Methyl oder durch R10 substituiertes Methyl oder Ethyl bedeutet,
R9 Wasserstoff bedeutet,
R10 Hydroxy, Carboxy oder -N(R11)R12 bedeutet, wobei
R11 Methyl und
R12 Methyl bedeutet, oder wobei
R11 und R12 zusammen und unter Einschluß des Stickstoffatoms, an das beide gebunden sind, einen Piperidinorest darstellen,
m die Zahl 2 oder 3 bedeutet,
n die Zahl 0 bedeutet,
p die Zahl 0 bedeutet und
q die Zahl 0 bedeutet,
und ihre Salze.

9. Verbindungen der Formel I nach Anspruch 1, in denen
R1 Wasserstoff bedeutet,
R2 Wasserstoff oder Fluor bedeutet,
R3 Wasserstoff bedeutet,
R4 Wasserstoff bedeutet,
R5 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin, Pyrimidin und Benzimidazol,
R7 Wasserstoff bedeutet,
R8 Wasserstoff, Methyl, Nitro, 1-4C-Alkoxycarbonyl oder durch R10 substituiertes Methyl oder Ethyl bedeutet,
R9 Wasserstoff bedeutet,
R10 Hydroxy, Carboxy oder -N(R11)R12 bedeutet, wobei
R11 Methyl und
R12 Methyl bedeutet, oder wobei
R11 und R12 zusammen und unter Einschluß des Stickstoffatoms, an das beide gebunden sind, einen Piperidinorest darstellen,
m eine Zahl von 2 bis 4 bedeutet,
n die Zahl 0 bedeutet,
p die Zahl 0 bedeutet und
q die Zahl 0 bedeutet,
und ihre Salze.

10. Verbindungen der Formel I nach Anspruch 1, in denen
R1 Wasserstoff bedeutet,
R2 Wasserstoff bedeutet,
R3 Wasserstoff bedeutet,
R4 Wasserstoff bedeutet,
R5 1-4C-Alkyl oder 1-4C-Alkoxy bedeutet,
R6 einen durch R8 und R9 substituierten Cyclus bedeutet, der ausgewählt ist aus der Gruppe bestehend aus Benzol, Thiazol, Imidazol, Triazol, Tetrazol, Thiadiazol, Pyridin und Pyrimidin,
R7 Wasserstoff bedeutet,
R8 Wasserstoff, Methyl oder durch R10 substituiertes Methyl oder Ethyl bedeutet,
R9 Wasserstoff bedeutet,
R10 Carboxy oder -N(R11)R12 bedeutet, wobei
R11 Methyl und
R12 Methyl bedeutet,
m eine Zahl von 2 bis 4 bedeutet,
n die Zahl 0 bedeutet,
p die Zahl 0 bedeutet und
q die Zahl 0 bedeutet,
und ihre Salze.

11. Verfahren zur Herstellung der Verbindungen der Formel I nach Anspruch 1, worin R1, R2, R3, R4, R5, R6, R7, m, n, p und q die in Anspruch 1 angegebenen Bedeutungen haben, und ihrer Salze, dadurch gekennzeichnet, daß man
a) Mercaptobenzimidazole der Formel II, 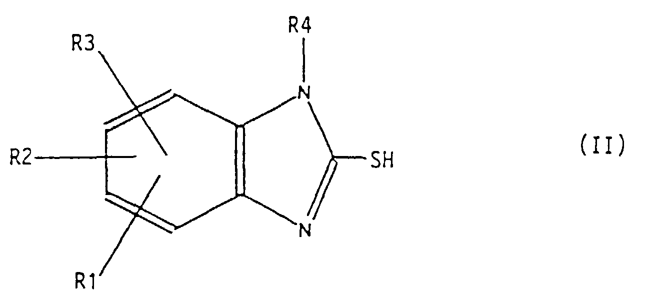 worin R1, R2, R3 und R4 die in Anspruch 1 angegebenen Bedeutungen haben, mit Picolinderivaten III, 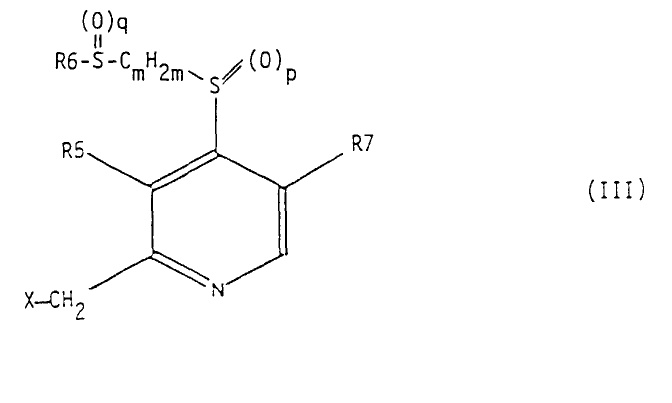 worin R5, R6, R7, m, p und q die in Anspruch 1 angegebenen Bedeutungen haben und X eine geeignete Abgangsgruppe darstellt, umsetzt oder daß man
b) Verbindungen der Formel IV, 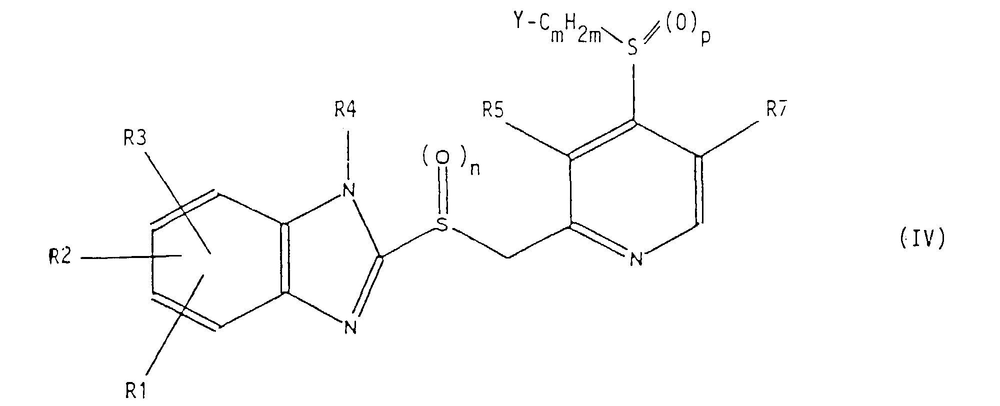 worin R1, R2, R3, R4, R5, R7 und m die in Anspruch 1 angegebenen Bedeutungen haben, n und p die Zahl 0 bedeuten und Y eine geeignete Abgangsgruppe darstellt, mit Thiolen R6-SH umsetzt und
(falls Verbindungen der Formel I mit n=1 bzw. p=1 und/oder q=1 die gewünschten Endprodukte sind), daß man anschließend die nach a) oder b) erhaltenen Verbindungen mit n=0 und/oder p=0 und/oder q=0 oxydiert, und/oder daß man erhaltene Verbindungen gewünschtenfalls anschließend in die Salze überführt und/oder daß man erhaltene Salze gewünschtenfalls anschließend in die freien Verbindungen überführt.

12. Arzneimittel enthaltend eine oder mehrere Verbindungen der Formel I nach Anspruch 1 und/oder ihre pharmakologisch verträglichen Salze.

13. Verbindungen der Formel I nach Anspruch 1 und/oder ihre pharmakologisch verträglichen Salze zur Anwendung bei der Bekämpfung von Helicobacter-Bakterien.

14. Verwendung von Verbindungen der Formel I nach Anspruch 1 und ihren pharmakologisch verträglichen Salzen zur Herstellung von Arzneimitteln für die Bekämpfung von Helicobacter-Bakterien.

## Claims

1. A compound of the formula I 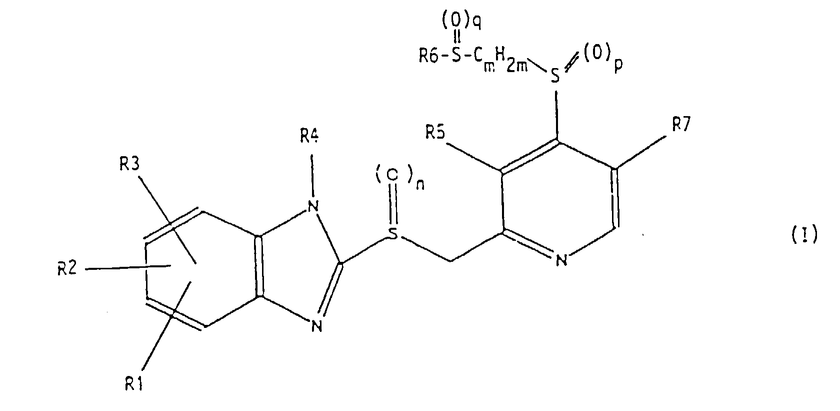 in which
R1 is hydrogen, 1-4C-alkyl or 1-4C-alkoxy,
R2 is hydrogen, 1-4C-alkyl, 1-4C-alkoxy, halogen, trifluoromethyl, 1-4C-alkoxy which is completely or predominantly substituted by fluorine, or chlorodifluoromethoxy or 2-chloro-1,1,2-trifluoroethoxy, or, together with R3, is 1-2C-alkylenedioxy which, if desired, is completely or partly substituted by fluorine, or chlorotrifluoroethylenedioxy,
R3 is hydrogen, 1-4C-alkoxy which is completely or predominantly substituted by fluorine, or chlorodifluoromethoxy or 2-chloro-1,1,2-trifluoroethoxy or, together with R2, is 1-2C-alkylenedioxy which, if desired, is completely or partly substituted by fluorine, or chlorotrifluoroethylenedioxy,
R4 is hydrogen or 1-4C-alkyl,
R5 is hydrogen, 1-4C-alkyl or 1-4C-alkoxy,
R6 is a cyclic or bicyclic radical which is substituted by R8 and R9 and is chosen from the group consisting of benzene, furan, thiophene, pyrrole, oxazole, isoxazole, thiazole, thiazoline, isothiazole, imidazole, imidazoline, pyrazole, triazole, tetrazole, thiadiazole, oxadiazole, pyridine, pyridine N-oxide, pyrimidine and benzimidazole,
R7 is hydrogen, 1-4C-alkyl or 1-4C-alkoxy,
R8 is hydrogen, 1-4C-alkyl, hydroxyl, 1-4C-alkoxy, halogen, nitro, carboxyl, 1-4C-alkoxycarbonyl, guanidino, 1-4C-alkyl which is substituted by R10, or -N(R11)R12,
R9 is hydrogen, 1-4C-alkyl, 1-4C-alkoxy, fluorine or trifluoromethyl,
R10 is hydroxyl, 1-4C-alkoxy, carboxyl, 1-4C-alkoxycarbonyl or -N(R11)R12, in which
R11 is hydrogen, 1-4C-alkyl or -CO-R13 and
R12 is hydrogen or 1-4C-alkyl, or in which
R11 and R12, together and including the nitrogen atom to which they are both bonded, are a piperidino or morpholino radical,
R13 is hydrogen, 1-4C-alkyl or 1-4C-alkoxy,
m is a number from 2 to 7,
n is the number 0 or 1,
p is the number 0 or 1 and
q is the number 0 or 1,
or one of its salts.

2. A compound of the formula I as claimed in claim 1, in which
R1 is hydrogen, 1-4C-alkyl or 1-4C-alkoxy,
R2 is hydrogen, 1-4C-alkyl, 1-4C-alkoxy, halogen, trifluoromethyl, 1-4C-alkoxy which is completely or predominantly substituted by fluorine, or chlorodifluoromethoxy or 2-chloro-1,1,2-trifluoroethoxy, or, together with R3, is 1-2C-alkylenedioxy which, if desired, is completely or partly substituted by fluorine, or chlorotrifluoroethylenedioxy,
R3 is hydrogen, 1-4C-alkoxy which is completely or predominantly substituted by fluorine, or chlorodifluoromethoxy or 2-chloro-1,1,2-trifluoroethoxy, or, together with R2, is 1-2C-alkylenedioxy which, if desired, is completely or partly substituted by fluorine, or chlorotrifluoroethylenedioxy,
R4 is hydrogen or 1-4C-alkyl,
R5 is hydrogen, 1-4C-alkyl or 1-4C-alkoxy,
R6 is a cyclic or bicyclic radical which is substituted by R8 and R9 and is chosen from the group consisting of benzene, furan, thiophene, pyrrole, oxazole, isoxazole, thiazole, thiazoline, isothiazole, imidazole, imidazoline, pyrazole, triazole, tetrazole, thiadiazole, oxadiazole, pyridine, pyridine N-oxide, pyrimidine and benzimidazole,
R7 is hydrogen, 1-4C-alkyl or 1-4C-alkoxy,
R8 is hydrogen, 1-4C-alkyl, hydroxyl, 1-4C-alkoxy, halogen, guanidino, 1-4C-alkyl which is substituted by R10, or -N(R11)R12,
R9 is hydrogen, 1-4C-alkyl, 1-4C-alkoxy, fluorine or trifluoromethyl,
R10 is hydroxyl, 1-4C-alkoxy, carboxyl, or -N(R11)R12, in which
R11 is hydrogen, 1-4C-alkyl or -CO-R13 and
R12 is hydrogen or 1-4C-alkyl, or in which
R11 and R12 , together and including the nitrogen atom to which they are both bonded, are a piperidino or morpholino radical,
R13 is hydrogen, 1-4C-alkyl or 1-4C-alkoxy,
m is a number from 2 to 7,
n is the number 0 or 1,
p is the number 0 or 1 and
q is the number 0 or 1,
or one of its salts.

3. A compound of the formula I as claimed in claim 1, in which R8 is nitro, carboxyl or 1-4C-alkoxycarbonyl, and/or in which R10 is 1-4C alkoxycarbonyl, or one of its salts.

4. A compound of the formula I as claimed in claim 1, in which
R1 is hydrogen,
R2 is hydrogen, halogen, methoxy, difluoromethoxy or trifluoromethyl,
R3 is hydrogen,
R4 is hydrogen,
R5 is hydrogen, 1-4C-alkyl or 1-4C-alkoxy,
R6 is a cyclic radical which is substituted by R8 and R9 and is chosen from the group consisting of benzene, isoxazole, thiazole, imidazole, triazole, tetrazole, thiadiazole, pyridine, pyridine N-oxide, and pyrimidine
R7 is hydrogen or 1-4C-alkyl,
R8 is hydrogen, 1-4C-alkyl, 1-4C-alkyl which is substituted by R10, or amino,
R9 is hydrogen or 1-4C-alkyl,
R10 is hydroxyl, carboxyl or -N(R11)R12, in which
R11 is hydrogen, 1-4C-alkyl or -CO-R13 and
R12 is hydrogen or 1-4C-alkyl, or in which
R11 and R12, together and including the nitrogen atom to which they are both bonded, are a piperidino radical,
R13 is hydrogen, 1-4C-alkyl or 1-4C-alkoxy,
m is a number from 2 to 4,
n is the number 0 or 1,
p is the number 0 and
q is the number 0,
or one of its salts.

5. A compound of the formula I as claimed in claim 1, in which
R1 is hydrogen,
R2 is hydrogen, halogen or methoxy,
R3 is hydrogen,
R4 is hydrogen,
R5 is hydrogen, 1-4C-alkyl or 1-4C-alkoxy,
R6 is a cyclic radical which is substituted by R8 and R9 and is chosen from the group consisting of benzene, isoxazole, thiazole, imidazole, triazole, tetrazole, thiadiazole, pyridine, pyridine N-oxide, pyrimidine and benzimidazole,
R7 is hydrogen or 1-4C-alkyl,
R8 is hydrogen, 1-4C-alkyl, hydroxyl, nitro, guanidino, carboxyl, 1-4C-alkoxycarbonyl, 1-4C-alkyl which is substituted by R10, or amino,
R9 is hydrogen or 1-4C-alkyl,
R10 is hydroxyl, carboxyl, 1-4C-alkoxycarbonyl or -N(R11)R12, in which
R11 is hydrogen, 1-4C-alkyl or -CO-R13 and
R12 is hydrogen or 1-4C-alkyl, or in which
R11 and R12, together and including the nitrogen atom to which they are both bonded, are a piperidino radical,
R13 is hydrogen, 1-4C-alkyl or 1-4C-alkoxy,
m is a number from 2 to 4,
n is the number 0 or 1,
p is the number 0 and
q is the number 0,
or one of its salts.

6. A compound of the formula I as claimed in claim 1, in which
R1 is hydrogen,
R2 is hydrogen or methoxy,
R3 is hydrogen,
R4 is hydrogen,
R5 is 1-4C-alkyl or 1-4C-alkoxy,
R6 is a cyclic radical which is substituted by R8 and R9 and is chosen from the group consisting of benzene, thiazole, triazole, tetrazole, thiadiazole and pyridine,
R7 is hydrogen,
R8 is hydrogen, 1-4C-alkyl, or methyl or ethyl which is substituted by R10,
R9 is hydrogen or 1-4C-alkyl,
R10 is hydroxyl, carboxyl or -N(R11)R12, in which
R11 is 1-4C-alkyl and
R12 is 1-4C-alkyl, or in which
R11 and R12, together and including the nitrogen atom to which they are both bonded, are a piperidino radical,
m is the number 2 or 3,
n is the number 0,
p is the number 0 and
q is the number 0,
or one of its salts.

7. A compound of the formula I as claimed in claim 1, in which
R1 is hydrogen,
R2 is hydrogen, fluorine or methoxy,
R3 is hydrogen,
R4 is hydrogen,
R5 is 1-4C-alkyl or 1-4C-alkoxy,
R6 is a cyclic radical which is substituted by R8 and R9 and is chosen from the group consisting of benzene, thiazole, imidazole, triazole, tetrazole, thiadiazole, pyridine, pyrimidine and benzimidazole,
R7 is hydrogen,
R8 is hydrogen, 1-4C-alkyl, hydroxyl, nitro, guanidino, carboxyl, 1-4C-alkoxycarbonyl, or methyl or ethyl which is substituted by R10,
R9 is hydrogen or 1-4C-alkyl,
R10 is hydroxyl, carboxyl or -N(R11)R12, in which
R11 is 1-4C-alkyl and
R12 is 1-4C-alkyl, or in which
R11 and R12, together and including the nitrogen atom to which they are both bonded, are a piperidino radical,
m is a number from 2 to 4,
n is the number 0,
p is the number 0 and
q is the number 0,
or one of its salts.

8. A compound of the formula I as claimed in claim 1, in which
R1 is hydrogen,
R2 is hydrogen,
R3 is hydrogen,
R4 is hydrogen,
R5 is 1-4C-alkyl or 1-4C-alkoxy,
R6 is a cyclic radical which is substituted by R8 and R9 and is chosen from the group consisting of benzene, thiazole, triazole, tetrazole, thiadiazole and pyridine,
R7 is hydrogen,
R8 is hydrogen, methyl, or methyl or ethyl which is substituted by R10,
R9 is hydrogen,
R10 is hydroxyl, carboxyl or -N(R11)R12, in which
R11 is methyl and
R12 is methyl, or in which
R11 and R12, together and including the nitrogen atom to which they are both bonded, are a piperidino radical,
m is the number 2 or 3,
n is the number 0,
p is the number 0 and
q is the number 0,
or one of its salts.

9. A compound of the formula I as claimed in claim 1, in which
R1 is hydrogen,
R2 is hydrogen or fluorine,
R3 is hydrogen,
R4 is hydrogen,
R5 is 1-4C-alkyl or 1-4C-alkoxy,
R6 is a cyclic radical which is substituted by R8 and R9 and is chosen from the group consisting of benzene, thiazole, imidazole, triazole, tetrazole, thiadiazole, pyridine, pyrimidine and benzimidazole,
R7 is hydrogen,
R8 is hydrogen, methyl, nitro, 1-4C-alkoxycarbonyl, or methyl or ethyl which is substituted by R10,
R9 is hydrogen,
R10 is hydroxyl, carboxyl or -N(R11)R12, in which
R11 is methyl and
R12 is methyl, or in which
R11 and R12, together and including the nitrogen atom to which they are both bonded, are a piperidino radical,
m is a number from 2 to 4,
n is the number 0,
p is the number 0 and
q is the number 0,
or one of its salts.

10. A compound of the formula I as claimed in claim 1, in which
R1 is hydrogen,
R2 is hydrogen,
R3 is hydrogen,
R4 is hydrogen,
R5 is 1-4C-alkyl or 1-4C-alkoxy,
R6 is a cyclic radical which is substituted by R8 and R9 and is chosen from the group consisting of benzene, thiazole, imidazole, triazole, tetrazole, thiadiazole, pyridine and pyrimidine,
R7 is hydrogen,
R8 is hydrogen, methyl, or methyl or ethyl which is substituted by R10,
R9 is hydrogen,
R10 is carboxyl or -N(R11)R12, in which
R11 is methyl and
R12 is methyl,
m is a number from 2 to 4,
n is the number 0,
p is the number 0 and
q is the number 0,
or one of its salts.

11. A process for the preparation of a compound of the formula I as claimed in claim 1, in which R1, R2, R3, R4, R5, R6, R7, m, n, p and q have the meanings given in claim 1, or of one of its salts, which comprises
a) reacting a mercaptobenzimidazole of the formula II 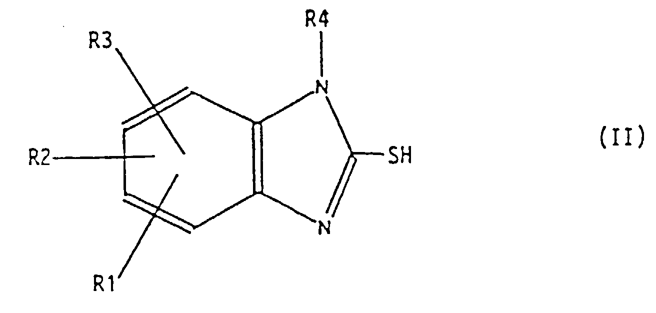 in which R1, R2, R3 and R4 have the meanings given in claim 1, with a picoline derivative III 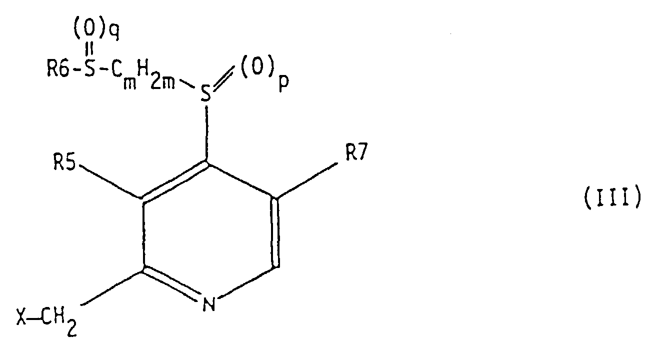 in which R5, R6, R7, m, p and q have the meanings given in claim 1 and X is a suitable leaving group, or
b) reacting a compound of the formula IV 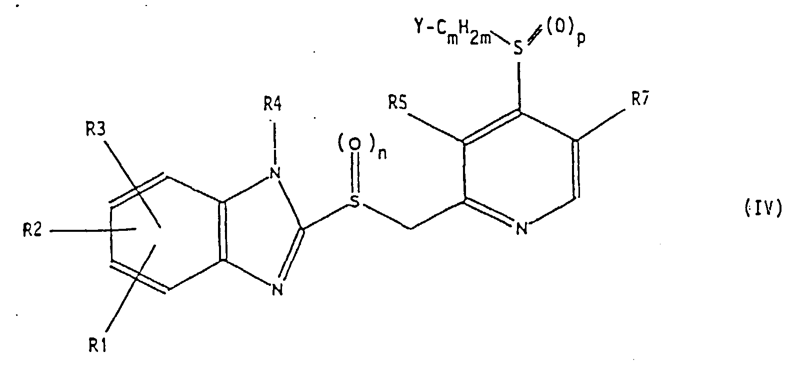 in which R1, R2, R3, R4, R5, R7 and m have the meanings given in claim 1, n and p are the number 0 and Y is a suitable leaving group, with a thiol R6-SH, and
(if a compound of the formula I where n=1 or p=1 and/or q=1 is the desired end product), subsequently oxidizing the compound obtained according to a) or b) where n=0 and/or p=0 and/or q=0, and/or if desired subsequently converting a compound obtained into a salt and/or if desired subsequently converting a salt obtained into the free compound.

12. A medicament comprising one or more compounds of the formula I as claimed in claim 1 and/or their pharmacologically tolerated salts.

13. A compound of the formula I as claimed in claim 1 and/or one of its pharmacologically tolerated salts for use in combating Helicobacter bacteria.

14. The use of a compound of the formula I as claimed in claim 1 or of one of its pharmacologically tolerated salts for the preparation of medicaments for combating Helicobacter bacteria.

## Revendications

1. Composés de formule (I) 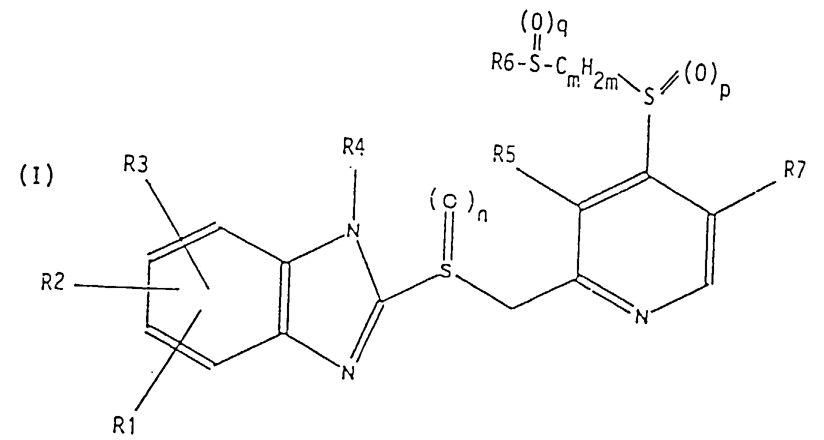 dans laquelle
R₁ représente un atome d'hydrogène, un groupe alkyle en C₁₋₄ ou alcoxy en C₁₋₄,
R₂ représente un atome d'hydrogène, un groupe alkyle en C₁₋₄, alcoxy en C₁₋₄, halogéno, trifluorométhyle, alcoxy en C₁₋₄ partiellement ou totalement fluoré, chlorodifluorométhoxy, 2-chloro-1,1,2-trifluoroéthoxy, ou représente en combinaison avec R₃ un groupe alkylènedioxy en C₁₋₂ partiellement ou totalement fluoré ou un groupe chlorotrifluoroéthylènedioxy,
R₃ représente un atome d'hydrogène, un groupe alcoxy en C₁₋₄ partiellement ou totalement fluoré, chlorodifluorométhoxy, 2-chloro-1,1,2-trifluoroéthoxy ou représente en combinaison avec R₂ un groupe alkylènedioxy en C₁₋₂ partiellement ou totalement fluoré ou un groupe chlorotrifluoroéthylènedioxy,
R₄ représente un atome d'hydrogène ou un groupe alkyle en C₁₋₄,
R₅ représente un atome d'hydrogène, un groupe alkyle en C₁₋₄ ou alcoxy en C₁₋₄,
R₆ représente un cycle simple ou double portant un substituant R₈ ou R₉, choisi parmi les cycles benzène, furane, thiophène, pyrrole, oxazole, isoxazole, thiazole, thiazoline, isothiazole, imidazole, imidazoline, pyrazole, triazole, tétrazole, thiadiazole, oxadiazole, pyridine, pyridine-N-oxyde, pyrimidine et benzimidazole,
R₇ représente un atome d'hydrogène, un groupe alkyle en C₁₋₄ ou alcoxy en C₁₋₄,
R₈ représente un atome d'hydrogène, un groupe alkyle en C₁₋₄, hydroxy, alcoxy en C₁₋₄, halogéno, nitro, carboxy, (alcoxy en C₁₋₄)-carbonyle, guanidino, alkyle en C₁₋₄ portant un substituant R₁₀, ou un groupe -N(R₁₁)R₁₂,
R₉ représente un atome d'hydrogène, un groupe alkyle en C₁₋₄, alcoxy en C₁₋₄, fluoro ou trifluorométhyle,
R₁₀ représente un groupe hydroxy, alcoxy en C₁₋₄, carboxy, (alcoxy en C₁₋₄)-carbonyle ou un groupe -N(R₁₁)R₁₂, où
R₁₁ représente un atome d'hydrogène ou un groupe alkyle en C₁₋₄ ou -CO-R₁₃ et
R₁₂ représente un atome d'hydrogène ou un groupe alkyle en C₁₋₄, ou
R₁₁ et R₁₂ représentent conjointement, avec l'atome d'azote auquel ils sont liés, un résidu pipéridino ou morpholino,
R₁₃ représente un atome d'hydrogène, un groupe alkyle en C₁₋₄ ou alcoxy en C₁₋₄,
m représente un nombre entre 2 et 7,
n vaut 0 ou 1,
p vaut 0 ou 1, et
q vaut 0 ou 1,
et les sels de tels composés.

2. Composés de formule (I) selon la revendication 1 dans laquelle
R₁ représente un atome d'hydrogène, un groupe alkyle en C₁₋₄ ou alcoxy en C₁₋₄,
R₂ représente un atome d'hydrogène, un groupe alkyle en C₁₋₄, alcoxy en C₁₋₄, halogéno, trifluorométhyle, alcoxy en C₁₋₄ partiellement ou totalement fluoré, chlorodifluorométhoxy, 2-chloro-1,1,2-trifluoroéthoxy, ou représente en combinaison avec R₃ un groupe alkylènedioxy en C₁₋₂ partiellement ou totalement fluoré ou un groupe chlorotrifluoroéthylènedioxy,
R₃ représente un atome d'hydrogène, un groupe alcoxy en C₁₋₄ partiellement ou totalement fluoré, chlorodifluorométhoxy, 2-chloro-1,1,2-trifluoroéthoxy ou représente en combinaison avec R₂ un groupe alkylènedioxy en C₁₋₂ partiellement ou totalement fluoré ou un groupe chlorotrifluoroéthylènedioxy,
R₄ représente un atome d'hydrogène ou un groupe alkyle en C₁₋₄,
R₅ représente un atome d'hydrogène, un groupe alkyle en C₁₋₄ ou alcoxy en C₁₋₄,
R₆ représente un cycle simple ou double portant un substituant R₈ ou R₉, choisi parmi les cycles benzène, furane, thiophène, pyrrole, oxazole, isoxazole, thiazole, thiazoline, isothiazole, imidazole, imidazoline, pyrazole, triazole, tétrazole, thiadiazole, oxadiazole, pyridine, pyridine-N-oxyde, pyrimidine et benzimidazole,
R₇ représente un atome d'hydrogène, un groupe alkyle en C₁₋₄ ou alcoxy en C₁₋₄,
R₈ représente un atome d'hydrogène, un groupe alkyle en C₁₋₄, hydroxy, alcoxy en C₁₋₄, halogéno, guanidino, alkyle en C₁₋₄ portant un substituant R₁₀, ou -N(R₁₁)R₁₂,
R₉ représente un atome d'hydrogène, un groupe alkyle en C₁₋₄, alcoxy en C₁₋₄, fluoro ou trifluorométhyle,
R₁₀ représente un groupe hydroxy, alcoxy en C₁₋₄, carboxy ou -N(R₁₁)R₁₂, où
R₁₁ représente un atome d'hydrogène ou un groupe alkyle en C₁₋₄ ou -CO-R₁₃ et
R₁₂ représente un atome d'hydrogène ou un groupe alkyle en C₁₋₄, ou
R₁₁ et R₁₂ représentent conjointement, avec l'atome d'azote auquel ils sont liés, un résidu pipéridino ou morpholino,
R₁₃ représente un atome d'hydrogène, un groupe alkyle en C₁₋₄ ou alcoxy en C₁₋₄,
m représente un nombre entre 2 et 7,
n vaut 0 ou 1,
p vaut 0 ou 1, et
q vaut 0 ou 1,
et les sels de tels composés.

3. Composés de formule (I) selon la revendication 1 dans laquelle R₈ représente un groupe nitro, carboxy, ou (alcoxy en C₁₋₄)-carbonyle, et/ou où R₁₀ représente un groupe (alcoxy en C₁₋₄)-carbonyle, ainsi que les sels de ces composés.

4. Composés de formule (I) selon la revendication 1, dans laquelle
R₁ représente un atome d'hydrogène,
R₂ représente un atome d'hydrogène ou d'halogène ou un groupe méthoxy, difluorométhoxy ou trifluorométhyle,
R₃ représente un atome d'hydrogène,
R₄ représente un atome d'hydrogène,
R₅ représente un atome d'hydrogène, un groupe alkyle en C₁₋₄ ou alcoxy en C₁₋₄,
R₆ représente un cycle portant un substituant R₈ ou R₉, choisi parmi les cycles benzène, isoxazole, thiazole, imidazole, triazole, tétrazole, thiadiazole, pyridine, pyridine-N-oxyde et pyrimidine,
R₇ représente un atome d'hydrogène ou un groupe alkyle en C₁₋₄,
R₈ représente un atome d'hydrogène, un groupe alkyle en C₁₋₄ ou alkyle en C₁₋₄ portant un substituant R₁₀, ou amino,
R₉ représente un atome d'hydrogène ou un groupe alkyle en C₁₋₄,
R₁₀ représente un groupe hydroxy, carboxy ou -N(R₁₁)R₁₂, où
R₁₁ représente un atome d'hydrogène ou un groupe alkyle en C₁₋₄ ou -CO-R₁₃ et
R₁₂ représente un atome d'hydrogène ou un groupe alkyle en C₁₋₄, ou
R₁₁ et R₁₂ représentent conjointement, avec l'atome d'azote auquel ils sont liés, un résidu pipéridino,
R₁₃ représente un atome d'hydrogène, un groupe alkyle en C₁₋₄ ou alcoxy en C₁₋₄,
m représente un nombre entre 2 et 4,
n vaut 0 ou 1,
p vaut 0, et
q vaut 0,
et les sels de tels composés.

5. Composés de formule (I) selon la revendication 1 dans laquelle
R₁ représente un atome d'hydrogène,
R₂ représente un atome d'hydrogène ou d'halogène ou un groupe méthoxy,
R₃ représente un atome d'hydrogène,
R₄ représente un atome d'hydrogène,
R₅ représente un atome d'hydrogène ou un groupe alkyle en C₁₋₄ ou alcoxy en C₁₋₄,
R₆ représente un cycle portant un substituant R₈ ou R₉, choisi parmi les cycles benzène, isoxazole, thiazole, imidazole, triazole, tétrazole, thiadiazole, pyridine, pyridine-N-oxyde, pyrimidine et benzimidazole,
R₇ représente un atome d'hydrogène ou un groupe alkyle en C₁₋₄,
R₈ représente un atome d'hydrogène, un groupe alkyle en C₁₋₄, hydroxy, nitro, guanidino, carboxy, (alkyle en C₁₋₄)-carbonyle, alkyle en C₁₋₄ portant un substituant R₁₀, ou un groupe amino,
R₉ représente un atome d'hydrogène ou un groupe alkyle en C₁₋₄,
R₁₀ représente un groupe hydroxy, carboxy, (alcoxy en C₁₋₄)-carbonyle ou -N(R₁₁)R₁₂, où
R₁₁ représente un atome d'hydrogène ou un groupe alkyle en C₁₋₄ ou -CO-R₁₃ et
R₁₂ représente un atome d'hydrogène ou un groupe alkyle en C₁₋₄, ou R₁₁ et R₁₂ représentent conjointement, avec l'atome d'azote auquel ils sont liés, un résidu pipéridino,
R₁₃ représente un atome d'hydrogène, un groupe alkyle en C₁₋₄ ou alcoxy en C₁₋₄,
m représente un nombre entre 2 et 4,
n vaut 0 ou 1,
p vaut 0, et
q vaut 0,
et les sels de tels composés.

6. Composés de formule (I) selon la revendication 1 dans laquelle
R₁ représente un atome d'hydrogène,
R₂ représente un atome d'hydrogène ou un groupe méthoxy,
R₃ représente un atome d'hydrogène,
R₄ représente un atome d'hydrogène,
R₅ représente un groupe alkyle en C₁₋₄ ou alcoxy en C₁₋₄,
R₆ représente un cycle portant un substituant R₈ ou R₉, choisi parmi les cycles benzène, thiazole, triazole, tétrazole, thiadiazole, et pyridine,
R₇ représente un atome d'hydrogène,
R₈ représente un atome d'hydrogène, un groupe alkyle en C₁₋₄, ou un groupe méthyle ou éthyle portant un substituant R₁₀,
R₉ représente un atome d'hydrogène ou un groupe alkyle en C₁₋₄,
R₁₀ représente un groupe hydroxy, carboxy ou -N(R₁₁)R₁₂, où
R₁₁ représente un groupe alkyle en C₁₋₄, et
R₁₂ représente un groupe alkyle en C₁₋₄, ou
R₁₁ et R₁₂ représentent conjointement, avec l'atome d'azote auquel ils sont liés, un résidu pipéridino,
m vaut 2 ou 3,
n vaut 0,
p vaut 0, et
q vaut 0,
et les sels de tels composés.

7. Composés de formule (I) selon la revendication 1, dans laquelle
R₁ représente un atome d'hydrogène,
R₂ représente un atome d'hydrogène ou de fluor ou un groupe méthoxy,
R₃ représente un atome d'hydrogène,
R₄ représente un atome d'hydrogène,
R₅ représente un groupe alkyle en C₁₋₄ ou alcoxy en C₁₋₄,
R₆ représente un cycle portant un substituant R₈ ou R₉, choisi parmi les cycles benzène, thiazole, imidazole, triazole, tétrazole, thiadiazole, pyridine, pyrimidine et benzimidazole,
R₇ représente un atome d'hydrogène,
R₈ représente un atome d'hydrogène, un groupe alkyle en C₁₋₄, hydroxy, nitro, guanidino, carboxy, (alcoxy en C₁₋₄)-carbonyle, ou un groupe méthyle ou éthyle portant un substituant R₁₀,
R₉ représente un atome d'hydrogène ou un groupe alkyle en C₁₋₄,
R₁₀ représente un groupe hydroxy, carboxy ou -N(R₁₁)R₁₂, où
R₁₁ représente un groupe alkyle en C₁₋₄, et
R₁₂ représente un groupe alkyle en C₁₋₄, ou
R₁₁ et R₁₂ représente conjointement, avec l'atome d'azote auquel ils sont liés, un résidu pipéridino,
m est un nombre compris entre 2 et 4,
n vaut 0,
p vaut 0, et
q vaut 0,
et les sels de tels composés.

8. Composés de formule (I) selon la revendication 1, dans laquelle
R₁ représente un atome d'hydrogène,
R₂ représente un atome d'hydrogène,
R₃ représente un atome d'hydrogène,
R₄ représente un atome d'hydrogène,
R₅ représente un groupe alkyle en C₁₋₄ ou alcoxy en C₁₋₄,
R₆ représente un cycle portant un substituant R₈ ou R₉, choisi parmi les cycles benzène, thiazole, triazole, tétrazole, thiadiazole et pyridine,
R₇ représente un atome d'hydrogène,
R₈ représente un atome d'hydrogène ou un groupe méthyle, ou un groupe méthyle ou éthyle portant un substituant R₁₀,
R₉ représente un atome d'hydrogène,
R₁₀ représente un groupe hydroxy, carboxy ou -N(R₁₁)R₁₂, où
R₁₁ représente un groupe méthyle, et
R₁₂ représente un groupe méthyle, ou
R₁₁ et R₁₂ représentent conjointement avec l'atome d'azote auquel ils sont liés, un résidu pipéridino,
m vaut 2 ou 3,
n vaut 0,
p vaut 0, et
q vaut 0,
et les sels de tels composés.

9. Composés de formule (I) selon la revendication 1, dans lesquels
R₁ représente un atome d'hydrogène,
R₂ représente un atome d'hydrogène ou de fluor,
R₃ représente un atome d'hydrogène,
R₄ représente un atome d'hydrogène,
R₅ représente un groupe alkyle en C₁₋₄ ou alcoxy en C₁₋₄,
R₆ représente un cycle portant un substituant R₈ ou R₉, choisi parmi les cycles benzène, thiazole, imidazole, triazole, tétrazole, thiadiazole, pyridine, pyrimidine et benzimidazole,
R₇ représente un atome d'hydrogène,
R₈ représente un atome d'hydrogène, un groupe méthyle, nitro, (alcoxy en C₁₋₄)-carbonyle ou un groupe méthyle ou éthyle portant un substituant R₁₀,
R₉ représente un atome d'hydrogène,
R₁₀ représente un groupe hydroxy, carboxy ou -N(R₁₁)R₁₂, où
R₁₁ représente un groupe méthyle, et
R₁₂ représente un groupe méthyle, ou
R₁₁ et R₁₂ représentent conjointement avec l'atome d'azote auquel ils sont liés, un résidu pipéridino,
m est un nombre compris entre 2 et 4,
n vaut 0,
p vaut 0, et
q vaut 0,
et les sels de tels composés.

10. Composés de formule (I) selon la revendication 1, dans lesquels
R₁ représente un atome d'hydrogène,
R₂ représente un atome d'hydrogène,
R₃ représente un atome d'hydrogène,
R₄ représente un atome d'hydrogène,
R₅ représente un groupe alkyle en C₁₋₄ ou alcoxy en C₁₋₄,
R₆ représente un cycle portant un substituant R₈ ou R₉, choisi parmi les cycles benzène, thiazole, imidazole, triazole, tétrazole, thiadiazole, pyridine et pyrimidine,
R₇ représente un atome d'hydrogène,
R₈ représente un atome d'hydrogène, un groupe méthyle ou un groupe méthyle ou éthyle portant un substituant R₁₀,
R₉ représente un atome d'hydrogène,
R₁₀ représente un groupe carboxy ou -N(R₁₁)R₁₂, où
R₁₁ représente un groupe méthyle, et
R₁₂ représente un groupe méthyle,
m est un nombre compris entre 2 et 4,
n vaut 0,
p vaut 0, et
q vaut 0,
et les sels de tels composés.

11. Procédé de préparation des composés de formule (I) selon la revendication 1 dans laquelle R₁, R₂, R₃, R₄, R₅, R₆, R₇, m, n, p et q ont la signification indiquée dans la revendication 1, et des sels de ces composés, caractérisé par le fait que
a) l'on fait réagir des mercaptobenzimidazoles de formule (II) 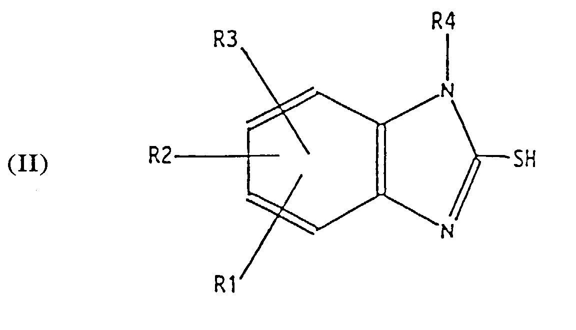 dans laquelle R₁, R₂, R₃ et R₄ ont la signification indiquée dans la revendication 1, avec des dérivés de picoline de formule (III), 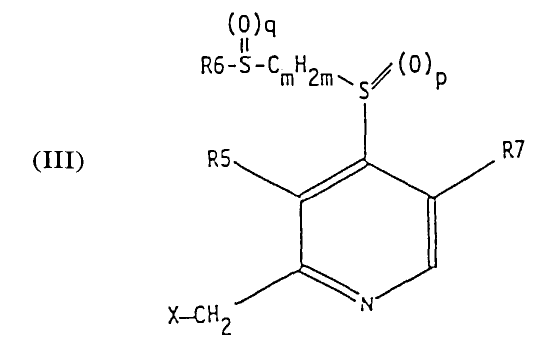 dans laquelle R₅, R₆, R₇, m, p et q ont la signification indiquée dans la revendication 1 et X représente un groupe partant approprié, ou
b) que l'on fait réagir des composés de formule (IV) 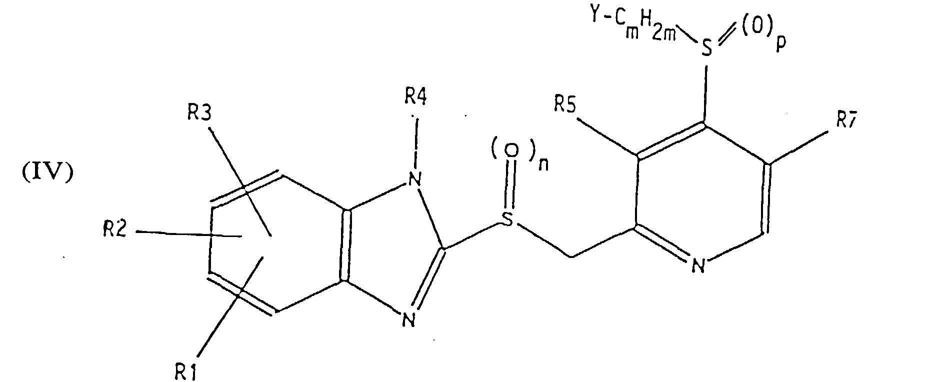 dans laquelle R₁, R₂, R₃, R₄, R₅, R₇ et m ont la signification indiquée dans la revendication 1, n et p valent 0 et Y représente un groupe partant approprié, avec un thiolène R₆-SH, et
(au cas où les produits finals recherchés sont des composés de formule (I) où n = 1 ou p = 1 et/ou q=1) en ce que l'on procède à l'oxydation des produits obtenus dans les étapes a) ou b) où n = 0 et/ou p = 0 et/ou p = 0, et/ou en ce que l'on convertit ensuite les composés obtenus en leur sels et/ou en ce que l'on convertit les sels obtenus éventuellement en les composés libres correspondants.

12. Médicament contenant un ou plusieurs composés de formule (I) selon la revendication 1 et/ou les sels pharmacologiquement acceptables de ces composés.

13. Composés de formule (I) selon la revendication 1 et/ou les sels pharmacologiquement acceptables de ceux-ci pour l'application dans le cadre de la lutte contre des bactéries de type héliobacter.

14. Utilisation de composés de formule (I) selon la revendication 1 et de leurs sels pharmacologiquement acceptables pour la préparation de médicaments pour la lutte contre des bactéries de type héliobacter.
